# EUROPEAN PATENT SPECIFICATION

(11) **EP 2 327 778 B1**
(45) Date of publication and mention of the grant of the patent: **20.05.2015**
(21) Application number: 09803065.3
(22) Date of filing: 03.08.2009
(51) Int. Cl.: C12N 15/09, C07K 16/08, C12N 1/15, C12N 1/19, C12N 1/21, C12N 5/10, C12P 21/02, C12Q 1/68, C07K 14/47, C07K 14/005

(54) **NUCLEIC ACID OCCURRING IN BLOOD FROM HEPATITIS PATIENT, POLYPEPTIDE ENCODED BY THE NUCLEIC ACID, AND USE OF THE SAME**
NUKLEINSÄURE AUS DEM BLUT VON HEPATITISPATIENTEN, VON DER NUKLEINSÄURE KODIERTES PEPTID UND VERWENDUNG
ACIDE NUCLÉIQUE SE PRÉSENTANT DANS LE SANG D'UN PATIENT ATTEINT D'UNE HÉPATITE, POLYPEPTIDE CODÉ PAR L'ACIDE NUCLÉIQUE ET SON UTILISATION

(30) Priority: 01.08.2008 JP 2008200050
(43) Date of publication of application: 01.06.2011
(73) Proprietor: Japanese Red Cross Society, Tokyo 105-8521 (JP)
(72) Inventor: SATOH, Kouei, Koga-shi Ibaraki 306-0204 (JP); TAKAKURA, Akiko, Tokyo 121-0836 (JP)
(74) Representative: Henderson, Helen Lee
(86) International application number: PCT/JP2009/063766
(87) International publication number: WO 2010/013830

(56) References cited:
- DATABASE UniProt [Online] 2005, AAVV: "Cryptococcus neoformans", XP002664763, retrieved from UniParc Database accession no. UPI000042E802
- DATABASE EMBL [Online] 14 October 2005 (2005-10-14), Copeland: "Pelobacter carbinolicus DSM2380, complete genome", XP002664764, Database accession no. CP00142
- DATABASE EMBL [Online] 11 March 2007 (2007-03-11), Fujiyama: "Macroporus eugenii", XP002664765, Database accession no. DE854115
- DATABASE EMBL [Online] 2 March 2007 (2007-03-02), Selvaraj: "Brassica napus", XP002664766, Database accession no. EE455776
- DATABASE EMBL [Online] 21 September 2005 (2005-09-21), NIH: "Danio rerio", XP002664767, Database accession no. DT865351
- DATABASE EMBL [Online] 10 August 2006 (2006-08-10), Leveque: "Chlorocebus aethiops", XP002664768, Database accession no. ED014909
- DATABASE EMBL [Online] 24 January 2006 (2006-01-24), Mueller: "Solanum lycopersicum", XP002664769, Database accession no. DU910975
- Laboratory guidelines for screening, diagnosis and monitoring of hepatic injury: In: "Laboratory Medicine Practice Guidelines", 2000, National Academy of Clinical Biochemistry, XP002664770, vol. 12, pages 1-60, * the whole document *
- LEARY T.P. ET AL.: 'Sequence and genomic organization of GBV-C: a novel member of the flaviviridae associated with human non-A- E hepatitis' J.MED.VIROL. vol. 48, no. 1, 1996, pages 60 - 7, XP000612659
- KAVIANI M.J. ET AL.: 'Occult hepatitis B virus infection and cryptogenic chronic hepatitis in an area with intermediate prevalence of HBV infection' WORLD J.GASTROENTEROL. vol. 12, no. 31, 2006, pages 5048 - 50, XP008144495

## Description

### TECHNICAL FIELD

The present invention relates to a novel nucleic acid found in blood of a hepatitis patient and a polypeptide encoded by the same. More specifically, the present invention relates a novel nucleic acid found out to exist at a high probability in a clinical example of hepatitis in which infection with any known hepatitis,virus is ruled out (hereinafter referred to as "hepatitis of unknown cause"), and a polypeptide encoded by the same. The present invention further relates to the use of the novel nucleic acid and the polypeptide encoded by the same.

### BACKGROUND ART

Hepatitis is an inflammation of the liver on account of various causes, and hepatitis is a medically important disease in which the liver function is damaged sometimes resulting in the death. Known causes of hepatitis include, for example, viruses, drugs, alcohol, and autoimmunity. In particular, a large number of patients exist suffering from viral hepatitis which is caused by a virus (hepatitis virus), and viral hepatitis is especially important because viral hepatitis is transmitted via blood and/or feces. Reported until now as hepatitis virus include those of the types of A, B, C, D, E, F, G, and TT. The relationship with hepatitis has been established in relation to the hepatitis viruses of the A, B, C, D, and E types. Many studies have been performed for these viruses. Serological and/or genetic detection methods have been established and are widely used for screening and diagnosis. In particular, screening is carried out in many countries for transfusion blood in relation to the B type and C type hepatitis viruses, which are blood-borne hepatitis viruses, which is useful to prevent the infection with the B type hepatitis and the C type hepatitis caused by the blood transfusion.

However, there is also a form of hepatitis, for which the cause or ethology has not been elucidated yet (Non-Patent Documents 1 and 2). Its diagnosis is problematic (Non-Patent Document 3). It is necessary to elucidate the cause of such hepatitis and establish a screening method, a diagnosis method, and a medical treatment method for such hepatitis.

### CITATION LIST

Non-Patent Documents:
Non-Patent Document 1: World J Gastroenterol. 2006 Aug 21; 12 (31) : 5048-50;
Non-Patent Document 2: Transplantation. 2000 Jul 27; 70(2): 292-7.
Non-Patent Document 3: Laboratory Medicine Practice Guidelines. 2000; 12:1-60

### SUMMARY OF THE INVENTION

### Problem to Be Solved by the Invention:

An object of the present invention is to identify a substance relevant to the hepatitis of unknown cause and provide information in relation thereto, especially gene information. Another object of' the present invention is to provide a method for detecting the substance and a material usable for the method.

### Solution to Problem:

The present inventors have extracted nucleic acids from 500 blood samples which are abnormal in alanine aminotransferase (ALT) / negative in hepatitis virus marker, and have performed the amplification by using a uniquely prepared helicase family-reactive helicase primer. As a result, it has been found that the amplification was successful with 12 samples. Sequence of the amplified nucleic acids were determined and run through nucleic acid sequence database. As a result, 8 samples were known nucleic acid sequences. However, 4 samples had no nucleic acid sequence found in the database. The sequences of 4 samples are identical with each other. The present inventors have carried out primer walking using the sample in which the novel sequence has been found out, and the have further clarified sequences in the 3' direction and the 5' direction of the gene including the novel sequence. Finally, a novel nucleic acid sequence (SEQ ID NO: 1) having a length of 9496 nucleotides has been obtained. As a result of the database retrieval, it has been confirmed that the obtained nucleic acid sequence is a novel sequence.

A method for detecting the presence of the novel gene in question has been devised on the basis of the sequence obtained as described above. Further, the sample was analyzed inter alia, in relation to the possibility/impossibility of reverse transcription, sensitivity to RNase and DNase, and restriction enzyme reaction. As a result, it has been revealed that this nucleic acid sequence exists as double strand DNA. According to the structural analysis for the double strand DNA thus revealed, putative open reading frame regions (SEQ ID NOS: 3 to 9) have been specified. The amino acid sequences (SEQ ID NOS: 10 to 17), which are encoded by these regions, have been revealed. Further, the novel DNA's in question are obtained for various clinical specimens, and nucleotide sequences thereof have been investigated in detail. As a result, it has been revealed that mutation occurs in the gene relatively rarely, and the sequence is conserved. Oligonucleotide useful to detect the concerning gene, have been designed.

Basis on the findings described above, the present invenion is summarized as follows:
1. A nucleic acid comprising the nucleotide sequence of SEQ ID NO: 1 or a nucleic acid comprising a nucleotide sequence complementary to the nucleotide sequence of SEQ ID NO: 1.
2. A primer or probe for detection of a nucleic acid according to item (1), the primer or probe including any one of the nucleotide sequences of SEQ ID NOS: 18 to 38.
3. A method for detecting a nucleic acid according to item (1), the method comprising using one or more primers or probes according to item (2).
4.An in vitro method of diagnosing hepatitis of unknown cause, the method comprising detecting a nucleic acid according to item (1), or a fragment thereof, in a sample obtained from a patient.
5. A polypeptide comprising an amino acid sequence encoded by the nucleotide sequence of nucleotides 3070 to 3669, 3826 to 4293, 5233 to 6117, 7198 to 7872, or 7888 to 8610 of SEQ ID NO: 1.
6. An antibody against the polypeptide of item (8),
7. An in vitro method of diagnosing hepatitis of unknown cause, the method comprising detecting a polypeptide according to item (8) or an antibody according to item (9).

The following items are disclosed by are not part of the invention claimed invention:
(1) A nucleic acid comprising a nucleotide sequence of an open reading frame encoding a protein having a length of not less than 20 amino acids in a nucleotide sequence of SEQ ID NO: 1.
(2) A polypeptide comprising an amino acid sequence encoded by the open reading frame of the nucleic acid or an amino acid sequence of a fragment thereof having a length of not less than 20 amino acids.
(3) A polynucleotide which has a nucleotide length of not less than 10 bases, which includes a partial nucleotide sequence of a nucleotide sequence of SEQ ID NO: 1 or a nucleotide sequence complementary to the nucleotide sequence, and which is specifically hybridizable to the nucleic acid.
(4) The polynucleotide as described above, wherein the polynucleotide has a nucleotide length of not more than 50 bases.
(5) An expression vector comprising the nucleic acid as described above.
(6) A cell which is transformed by introducing the nucleic acid as described above and which expresses a polypeptide encoded by the nucleic acid.
(7) A method for producing the polypeptide, comprising culturing the cell as described above and recovering the polypeptide from a culture.

In this specification, the term "nucleic acid" is used interchangeably with "gene", "DNA", "polynucleotide", and "oligonucleotide" in some cases. That is, the "nucleic acid" refers to the general nucleic acid which includes DNA and RNA. The term "polypeptide" is used interchangeably with "protein" and "peptide".

The term "hepatitis of unknown cause" refers to the hepatitis for which the infection with any known hepatitis virus is denied. More specifically, the term "hepatitis of unknown cause" refers to the hepatitis which is negative for the virus markers for the A type, B type, and C type hepatitis viruses as main causes of hepatitises although it is clinically diagnosed that any hepatitis occurs.

### EFFECT OF THE INVENTION

According to the present invention, a nucleic acid relevant to the hepatitis of unknown cause is provided. The detection of the nucleic acid of the present invention or of a translation product thereof is useful to diagnose the hepatitis of unknown cause.

### BRIEF DESCRIPTION OF THE DRAWINGS

Fig. 1 shows sequences of helicase family primers used to perform the screening for unknown genes and positions for setting the primers in Example.
Fig. 2 shows a sequence extending method used to determine a sequence of an unknown gene in Example.
Fig. 3 shows a procedure of a nucleic acid measuring method in Example.
Fig. 4 shows an arrangement of deduced ORF's in a nucleotide sequence of SEQ ID NO: 1.
Fig. 5 shows the construction of an expression construct.

### DESCRIPTION OF EMBODIMENTS

The nucleic acid of the present invention is found in blood of a patient of hepatitis which has ALT abnormality and/or which is negative for the hepatitis virus marker, i.e., hepatitis of unknown cause or unknown etiology. The nucleic acid of the present invention is composed of the nucleotide sequence of SEQ ID NO: 1 or the nucleotide sequence complementary thereto. The nucleic acid of the present invention is any one of a double strand and single strand.

The following are achieved on the basis of the nucleotide sequence described herein. That is, it is possible to identify a candidate open reading frame in said sequence, it is possible to predict sequences of proteins encoded by structural and nonstructural gene candidates encoded by the novel gene on the basis of.the information thereof, and it is possible to synthesize the protein or proteins (hereinafter referred to as "gene product" or "gene products") encoded by the structural and/or nonstructural gene candidate or candidates and a fragment or fragments thereof chemically or by means of the genetic engineering. The methods for identifying the open reading frame candidate from the sequence and the method for expressing the gene product encoded by the gene and the fragment thereof by means of genetic engineering or chemically synthesizing the same are well-known in the art. Therefore, described are also the fragment of the nucleic acid, the structural and nonstructural genes included in the nucleic acid, and the gene products thereof.

The structural and nonstructural genes are exemplified by a nucleic acid composed of a nucleotide sequence of an open reading frame encoding a protein having a length of not less than 20 amino acids in the nucleotide sequence of SEQ ID NO: 1. The gene product is exemplified by a polypeptide composed of an amino acid sequence encoded by the open reading frame of the nucleic acid or an amino acid sequence of a fragment thereof having a length of not less than 20 amino acids. The length of not less than 20 amino acids is usually a length sufficient to exhibit antigenicity.

More specifically, the structural and nonstructural genes are exemplified by a nucleic acid composed of any one of nucleotide sequences of SEQ ID NOS: 2 to 9. The gene product is exemplified by a polypeptide composed of any one of amino acid sequences of SEQ ID NOS: 10 to 17.

It is known that any mutation, which does not affects the function, may exists in a gene. Therefore, it is acknowledged that the nucleic and amino acids described herein may include not only those having the sequences exemplified but also those composed of nucleotide sequences and amino acid sequences substantially having the same scope. This can be judged by the ratio of the homology of a sequence that is readily distinguishable when the nucleotide sequence or amino acid sequence in question is compared with another sequence (for example, any known gene of hepatitis cause). The substantially same scope can be determined with reference to the diversity of the sequence in a species known in relation to known organisms of the same species. For example, it is possible to clarify the taxonomical position of an organism from which the gene originates, by comparing the novel gene sequence with known gene sequences. The comparison between the novel gene and known gene sequences can be easily carried out by utilizing a database (for example, AAA) publicly available.

A sequence, which is substantially the same as a nucleotide sequence of the open reading frame encoding the protein having the length of not less than 20 amino acids in relation to the nucleotide sequence of SEQ ID NO: 1 (for example, SEQ ID NOS: 1 to 9), is, for example, a sequence having the identity of not less than about 95% preferably not less than 97% with respect to any one of the nucleotide sequences mentioned above.

Further, nucleotide sequences, which code for the amino acid sequences of SEQ ID NOS: 10 to 17 encoded by the nucleotide sequences described above (for example, nucleotide sequences of SEQ ID NOS: 2 to 9) respectively, in accordance with the degeneration of the genetic code, are also included in the "substantially same nucleotide sequence".

An amino acid sequence, which is substantially the same as the amino acid sequence encoded by the open reading frame of the nucleic acid or the amino acid sequence of the fragment thereof having the length of not less than 20 amino acids (for example, SEQ ID NOS: 10 to 17), is, for example, a sequence in which any amino acid in the sequence is substituted by the substitution between amino acids known not to greatly change the structure of the protein. The substitution as described above usually includes the interchangeable substitution between aliphatic amino acids Ala, Val, Leu, and Ile, the interchangeable substitution between Ser and Thr, the interchangeable substitution between Asp and Glu, the interchangeable substitution between Asn and Gln, the interchangeable substitution between Lys and Arg, and the interchangeable substitution between Phe and Tyr.

Owing to the provision of the nucleic acid of the present invention, it is possible to find out sequences specific to the nucleic acid of the present invention (especially the novel genes included in the nucleic acid), it is possible to synthesize oligonucleotides having the sequences and/or sequences complementary thereto, and it is also possible-to detect and/or amplify the nucleic acid of the present invention by using the same as primers and/or probes. The region, which is specific to the sequence, can be easily found out in practice by utilizing the database as described above and any commercially available software (for example, Lasergene DNASTAR (trade name)). Further, it is also possible to specify the primer and/or probe suitable for the detection and/or the amplification of the gene by using any software in the same manner as described above. The method for constructing a gene detecting system by using the primer and/or probe as described above is well-known in the art. Reference may be made, for example, to "PCR Modern Utilization Manual (written by Hiromi Sasaki, Yodo-sha, 2003). In the present invention, the polymerase chain reaction (Polymerase Chain Reaction: PCR) is used as the method for amplifying the gene by way of example. However, it is also possible to use any amplification method other than PCR, for example, the LCR (Ligase Chain Reaction) method. The two-stage PCR is also included in PCR.

The primer/probe as described above is exemplified by a polynucleotide which has a nucleotide length of not less than 10 bases, preferably not less than 15 bases, and especially preferably not less than 20 bases, which includes a partial nucleotide sequence of the nucleotide sequence of SEQ ID NO: 1 or any nucleotide sequence complementary to the nucleotide sequence, and which is specifically hybridizable to the nucleic acid of the present invention. The condition of the hybridization may be any known condition. However, the condition is usually a hybridization condition usable in a protocol of the typical southern blotting as follows.
1. DNA is transferred from a gel after agarose gel electrophoresis, and nitrocellulose, on which DNA is immobilized, is immersed in a 3 x SSC solution to perform a treatment at 65°C for 30 minutes.
2. The sample is immersed in a 5 x SSC, 1% SDS, 1 x denhardt solution (containing labeled probe) to perform a treatment at 65°C for 2 hours.
3. The temperature is further held at 65°C overnight.
4. The sample is immersed in about 100 mL of a 2 x SSC, 0.1% SDS solution, followed by being permeated at room temperature.
5. The solution is exchanged with a 0.2 x SSC, 0.1% SDS solution, the temperature is held at 65°C for 30 minutes, and the solution is exchanged again to hold the temperature.
6. The sample is washed with 2 x SSC and dried in air, followed by autoradiography.

The polynucleotide described above usually has a nucleotide length of not more than 50 bases, preferably not more than 40 bases, and especially preferably not more than 30 bases.

Examples of the polynucleotide described above are specifically exemplified by those including the nucleotide sequences of SEQ ID NOS: 18 to 38.

Examples of the primer combination for PCR are exemplified by SEQ ID NOS: 18 and 19, SEQ ID NOS: 19 and 20, SEQ ID NOS: 22 and 23, SEQ ID NOS: 24 and 25, SEQ ID NOS: 26 and 27, SEQ ID NOS: 28 and 29, SEQ ID NOS: 30 and 31, SEQ ID NOS: 32 and 33, SEQ ID NOS: 34 and 35, SEQ ID NOS: 36 and 37, and SEQ ID NOS: 38 and 39. Examples of the primer combination for the two-stage PCR are exemplified by a combination of SEQ ID NOS: 18 and 19 for the first amplification and a combination of SEQ ID NOS: 19 and 20 for the second amplification.

As described in the Examples, it has been clarified that a mutation occurs extremely rarely in the nucleic acid of the present invention, wherein the sequence conservation is extremely high. Therefore, it is possible for those skilled in the art to easily understand that primer combinations, which have an equivalent performance, exist other than the primer combinations specifically disclosed in this specification.

An expression vector is also described in which the nucleic acid of the present invention or a part thereof is incorporated. In this specification, the term "expression vector" means the vector which inserts an exogenous polynucleotide into a host cell gene in an expressible state. Specifically, the expression vector is the vector which has a control sequence capable of incorporating the polynucleotide. The term "part" means the portion which encodes a peptide sufficient to allow a gene product to function or sufficient to exhibit the antigenicity.

The expression vector can be effectively used to obtain a virus peptide having the immunological activity or the biological activity. At present, various vectors are known as the vector for incorporating a gene desired to be expressed. Further, various host cells are also known as the host cell into which the gene is incorporated by using the vector to finally express the peptide.

The expression vector is usually a recombinant expression vector which has the nucleotide sequence of the nucleic acid of the present invention or ORF thereof, wherein the ORF is connected to be operable with respect to the control sequence compatible to a desired host in the recombinant expression vector. A recombinant gene expression system can be constructed by using the expression vector.

In this specification, the term "transformed cell" means the cell capable of expressing all or a part of the polypeptide encoded by the nucleic acid of the present invention or a part thereof.

The transformed cell can be obtained by transforming a host cell by directly introducing the polynucleotide including all or a part of the nucleic acid of the present invention or by introducing the recombinant expression vector incorporated with the polynucleotide into the host cell. It is possible to use a known transfer vector and a host cell.

The polypeptide, which is encoded by the nucleic acid of the present invention, can be produced by culturing the host cell as described above and recovering the expressed polypeptide from a culture. The culture condition for the host cell is appropriately selected, depending on the host cell to be used. The condition for recovering the polypeptide is appropriately selected depending on the property of the object polypeptide.

When the gene product encoded by the novel gene or the fragment thereof can be once obtained, the gene product or the fragment thereof can be used as the antigen to detect and/or separate the antibody against the gene product or the fragment thereof existing in a biological sample. The method for detecting and/or separating the antibody against the antigen, which is based on the use of the antigen, is well-known in the technical field concerned.

The gene product or the fragment thereof can be also used to prepare and/or purify the antibody against the gene product or the fragment thereof. Various methods are available to prepare or purify the antibody by using the polypeptide including the gene product or the fragment thereof as the immune antigen or the probe, which are well-known in the technical field concerned.

When the fragment of the gene product is used as the immune antigen, it is desired that the fragment contains the epitope of the gene product. A large number of methods are disclosed to specify the position of the epitope existing in the polypeptide. It is also possible to seek out an epitope candidate from the elucidated amino acid sequence by using a commercially available software. The technique for analyzing and determining the epitope is well-known in the concerning technical field, and any analysis tool is commercially supplied.

The present invention will be explained more specifically below in accordance with the Examples. However, the present invention is not limited to the Examples. Unless otherwise specified, Examples shown below were carried out in accordance with the standard method (see, for example, Sambrook et al., MOLECULAR CLONING, A LABORATORY MANUAL, 2nd Ed; Cold Spring Harbor Laboratory Press, Cold Spring Harbor, New York (1989)).

### Example 1

### <1> Settling and preparation of primers for detecting helicase gene

It was thought very likely that an unknown hepatitis pathogen existed in blood which screened negative for the known B type hepatitis virus and the C type hepatitis virus, even though the ALT value was abnormal (which is the marker for indicating the liver function and which is used as one of the screening tests for transfusion bloods). Accordingly, blood, which was abnormal in ALT and which was negative for the hepatitis virus marker, was selected as a starting material to obtain the unknown hepatitis pathogen.

A helicase family common primer was selected as a tool for seeking out the gene of a novel hepatitis pathogen candidate from the blood, and the primer was prepared and used. The reason is that it is known that a protein, responsible for a function essential for life, exists commonly to many organisms, and the amino acid sequence thereof (and hence the nucleic acid sequence encoding the same) is conserved. Such a protein is also expected to exist in the objective novel hepatitis pathogen candidate gene. The following facts are known. That is, helicase is an enzyme that converts the double strand structure of DNA/DNA or DNA/RNA or the secondary structure of RNA into a single strand by cutting or cleaving hydrogen bonds. Helicase is one of the enzymes essential for the existence of the organism and has a role, in the replication of DNA, the repair, the recombination, the transcription of RNA, the splicing, and the translation into protein etc; helicase is known to exist in many organisms. In view of the above, the primer, which had the nucleic acid sequence common to the helicase family, was selected as the tool to be used to seek out the objective novel hepatitis pathogen gene. Sequence information was collected by screening the helicase family, and the sequence characteristics were investigated. Subsequently, the nucleic acid sequence of helicase of the virus was used as the basis, and inosine insertion was performed therefor to prepare or manufacture helicase primers reactive with a wide range of helicase family genes.

That is, the primers for detecting the helicase gene to be used were prepared by performing inosine insertion with respect to the sequence selected from the NS3 helicase consensus domain of the HCV helicase family. Specifically, the following three types of primers were prepared and used.
IA-3 (forward primer): CCI ACI ggI AgI ggI AAR AgC AC (SEQ ID NO: 40);
IV-3 (reverse primer): CTI CCM gTg CgI CCI CgS CgY Tg (SEQ ID NO: 41);
III R-2 (forward primer): CCI ggR IIg TIg CIg TRg C (SEQ ID NO: 42).

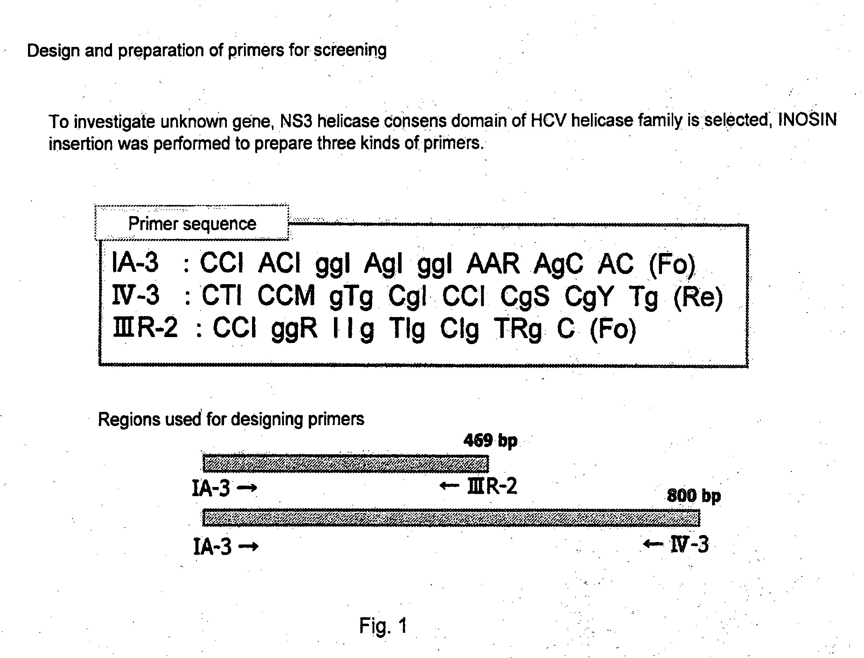

It was postulated that a gene fragment of 469 bp would be amplified from a combination of IA-3 and III R-2 and a gene fragment of 800 bp would be amplified from a combination of IA-3 and IV-3 (Fig. 1).

### <2> Amplification and analysis of unknown pathogen candidate gene fragment

It was tried to amplify the helicase gene (gene fragment of unknown pathogen candidate) from 500 samples of ALT abnormal human blood specimens by using the primers for detecting the helicase gene.

Nucleic acid was extracted from the specimen by using ExR&D Smitest Kit (Medical & Biological Laboratories Co., Ltd.). 100 µl of blood plasma obtained from the specimen blood was introduced into a centrifuge tube to which 15 µL of Solution I (enzyme solution), 380 µL of Solution II (specimen diluent solution), and 5 µL of Solution IV (coprecipitation reagent solution) of the kit were added, followed by incubation at 55°C for 30 minutes. Subsequently, 250 µL of Solution III (protein dissolvent solution) was added and incubated at 55°C for 15 minutes. Then 600 µL of isopropanol was added and incubated in ice for 10 minutes. After the completion of incubation, centrifugation was performed at 15,000 rpm for 10 minutes to obtain a precipitate while discarding the supernatant. 600 µL of 75% (V/v) ethanol was added thereto. The solution was centrifuged again at 15,000 rpm for 10 minutes, and the obtained precipitate was dried. 30 µL of distilled water (DW) was added to the dried precipitate so that the precipitate was dissolved, and the solution obtained was provided as template DNA.

In order to amplify the helicase gene (unknown pathogen gene), the polymerase chain reaction (PCR), which was based on the use of TaKaRa Ex-Taq as an enzyme and which used the template DNA as a template, was performed by using a TaKaRa PCR Thermal Cycler 480 amplifier. The PCR reaction solution contained the followings per tube: 20 µL of template DNA, 0.25 µL of Ex-Taq, 20 µL of 10 x buffer, 1 µL of 10 mM dNTP, 0.5 µL of forward primer IA-3, 0.5 µL of reverse primer IV-3, and 22.75 µL of distilled water (total amount: 50 µL). The reaction condition of PCR was as follows. The amplification cycle consisted of denaturation at 94°C for 30 seconds, annealing at 55°C for 30 seconds, and extension at 72°C for 2 minutes. This was performed 35 times after the denaturation reaction at 94°C for 2 minutes, and was followed by the extension at 72°C for 7 minutes.

The amplified gene was separated by means of agarose gel electrophoresis (gel concentration: 1.5%, 20 mA, 30 minutes), the gene was thereafter stained with ethidium bromide for 30 minutes, and the gene was detected by being irradiated with ultraviolet light. Twelve samples, in which the helicase gene (unknown pathogen candidate gene) was strongly amplified, were found out from 500 samples of the ALT abnormal human blood specimens.

Subsequently, the amplified nucleic acid was excised from the gel of the electrophoresis and purified by using MinElute Gel Extraction kit (Qiagen). Specifically, the weight of the gel was firstly measured. Buffer QG provided with the kit was added in an amount of 600 µL per 100 mg of the gel, followed by incubation at 50°C for 10 minutes. After the completion of incubation, isopropanol, which was in a 1/10 amount (v/v) of the solution, was added thereto, which was added to a MinElute Spin column to perform centrifugation at 15,000 rpm for 1 minute. Subsequently, 500 µL of Buffer QG was added to the column, followed by centrifugation at 15,000 rpm for 1 minute to wash impurities. Subsequently, 700 µL of Buffer PE was added, followed by centrifugation at 15,000 rpm for 1 minute to repeat the washing. A further centrifugation was performed at 15,000 rpm for 1 minute without adding any buffer to completely remove the washing solution from the column. After that, a tube was set under the column in order to receive the eluted matter. 10 µL of Buffer EB was added, followed by centrifugation at 15,000 rpm for 1 minute to elute the nucleic acid which was recovered in the tube disposed thereunder.

Subsequently, the sequence of the recovered amplified nucleic acid was determined in accordance with the following procedure. First, the amplified nucleic acid was amplified by using Big Dye Terminator v1.1 cycle sequencing kit (ABI), GeneAmp PCR system 9700 (Applied Biosystems), and two types of (forward and reverse) amplification solutions having the following component compositions: forward (8 µL of Pre Mix, 4 µL of forward primer IA-3, 4 µL of template (nucleic acid), and 4 µL of distilled water) and reverse (8 µL of Pre Mix, 4 µL of reverse primer IV-3, 4 µL of template (nucleic acid), and 4 µL of distilled water). Subsequently, a reaction product (amplified nucleic acid) was purified by using DyeEx 2.0 Spin kit (Qiagen). Specifically, a DyeEx 2.0 Spin column provided with the kit was centrifuged at 3,200 rpm for 3 minutes, and then the total amount of the reaction product was added to the column, followed by centrifugation at 3,200 rpm for 3 minutes to purify the reaction product. The purified amplified nucleic acid (liquid) was treated at 95°C for 2 minutes. The sample was placed on ice (0°C) for 10 minutes, followed by being applied to centrifugation, and then the total amount thereof was transferred to a sequence-measuring plate. The plate was analyzed by ABIPRISM 3100 Gene tic Analyzer by using Gene tic Analyzer with EDTA 3100 POP-6 polymer, and the sequence was determined. In the sequence determination, DT3100POP6 (BD) v2.mob was used as Mobility File, SR-Seq50-POP6-20S-5400sec was used as Run module, and BC-3100SR-SeqOffFtOff was used as Analysis module. The obtained data was analyzed by using an analysis software SEQUENCER GENETYX-MAC.

The homology screening was performed by using DDBJ BLAST and NCBI BLAST. As for the sequence, the homology screening was firstly performed. The open reading frame analysis, the translation into the amino acid sequence, the translation into the amino acid sequence after performing the complementary conversion, the homology screening for the amino acid sequence, the restriction enzyme site analysis, and the GC content distribution analysis were carried out all samples having any possibility of containing an unknown pathogen candidate gene. As a result, it was revealed that the amplified sequences were sequences of bacteria and G type hepatitis virus (i.e., known pathogen sequences) for 8 samples of 12 sample which exhibited the strong positive result in the amplification based on the use of the helicase common primer. The remaining 4 samples all contained the same sequence, for which it was revealed that the homology screening score (bits) was not more than 50, the E value was 3.5, so the sequence had a high possibility of being a novel sequence. Therefore, it was intended to clarify the entire sequence by subsequently performing the extension of the gene.

### <3> Determination of sequence of unknown pathogen candidate gene

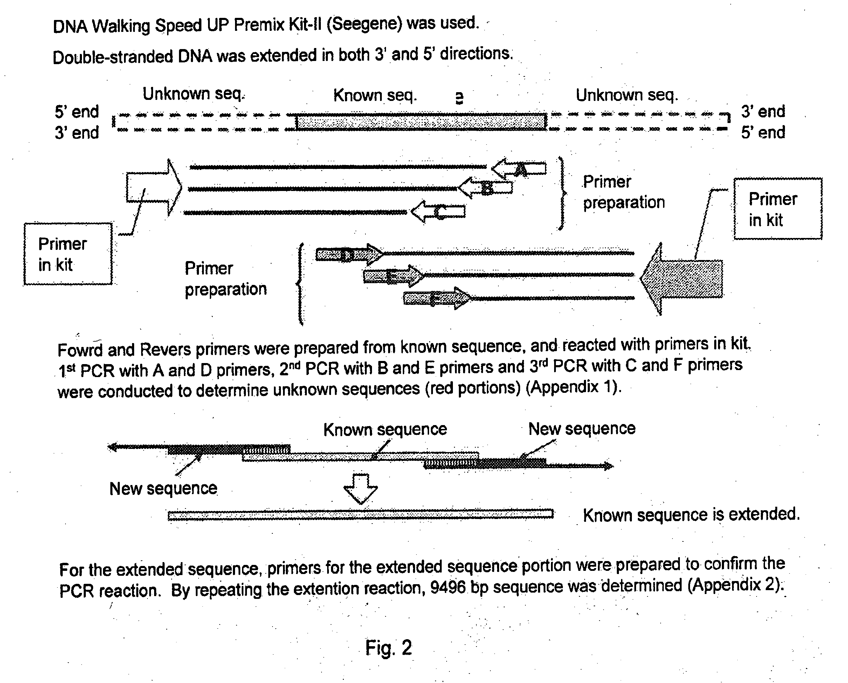

Fig. 2 shows an outline of the primer walking method by which the gene sequence of the present invention was determined. A plurality of forward primers (A, B, C) and reverse primers (D, E, F) were manufactured on the basis of the gene sequence of the unknown pathogen candidate determined in accordance with <2>. The primers were used in accordance with an explanatory pamphlet for the handling appended to the kit together with DNA Walking Speed UP Premix Kit-II (Seegene) to determine the undetermined gene sequence of the unknown pathogen candidate (indicated as unknown Seq in Fig. 2). Specifically, Primer 1 and Primer A provided with the kit were used to carry out the nucleic acid (DNA) amplification by using, as the template, the nucleic acid fraction in which the unknown pathogen candidate gene fragment was amplified (1st PCR). Subsequently, DNA amplified in the 1st. PCR was used as the template to perform the 2nd DNA amplification (2nd PCR) by using Primer 2 and Primer B provided with the kit. Further, DNA amplified in 2nd PCR was used as the template to perform the 3rd DNA amplification (3rd PCR) by using Primer 3 and Primer C provided with the kit. DNA's obtained in the respective amplification processes were gel-purified to determine the sequences of the amplified nucleotides amplified by the sequencing operation as described above. This operation was repeatedly carried out until no new sequence was determined, so as to determine the entire nucleic acid sequence of the unknown pathogen candidate gene (SEQ ID NO: 1, hereinafter referred to as "nucleic acid sequence of the invention"). The compositions of the reaction solutions and the reaction conditions of 1st PCR, 2nd PCR, and 3rd PCR were as follows.

**Table 1**

| 1st PCR | |
|---|---|
| Four tubes were prepared for one specimen. | |
| Composition of PCR solution: | |
| Template DNA: | 5 µL |
| DW2-ACP (1-4) : | 2 µL |
| 1 or A: | 1 µL |
| Distilled water: | 2 µL |
| 2 x seeAmp Acp MasterMix: 10 µL | |
| total | 20 µL |

| PCR condition: | |
|---|---|
| 94°C: | 5 minutes |
| 42°C: | 1 minute |
| 72°C: | 2 minutes |
| Cycle of 94°C (0.5 minute) - 60°C (0.5 minute) - 72°C (100 seconds): 30 times | |
| 72°C: | 7 minutes |

| 2nd PCR | |
|---|---|
| Composition of PCR solution: | |
| 1st PCR product: | 4 µL |
| DW2-ACP (1-4) : | 2 µL |
| 2 or B: | 1 µL |
| Distilled water: | 2 µL |
| MasterMix: 10 µL | |
| total | 20 µL |
| PCR condition: | |
| 944°C : | 3 minutes |
| Cycle of 94°C (0.5 minute) - .60°C (0.5 minute) - 72°C (1 minute): 30 times | |
| 72°C: | 7 minutes |

| 3rd PCR | |
|---|---|
| Composition of PCR solution: | |
| 50-fold solution of 2nd PCR product: | 5 µL |
| Uni P2: | 1 µL |
| 3 or C: | 1 µL |
| Distilled water: | 3 µL |
| 2 x seeAmp Acp MasterMix: 10 µL | |
| total | 20 µL |
| PCR condition: | |
| 94°C: | 3 minutes |
| Cycle of 94°C (0.5 minute) - 60°C (0.5 minute) - 72°C (1 minute): 30 times | |
| 72°C: | 7 minutes |

| PCR condition: | |
|---|---|
| 94°C: | 3 minutes |
| Cycle of 94°C (0.5 minute) - 60°C (0.5 minute) - 72°C (1 minute): 30 times | |
| 72°C: | 7 minutes |

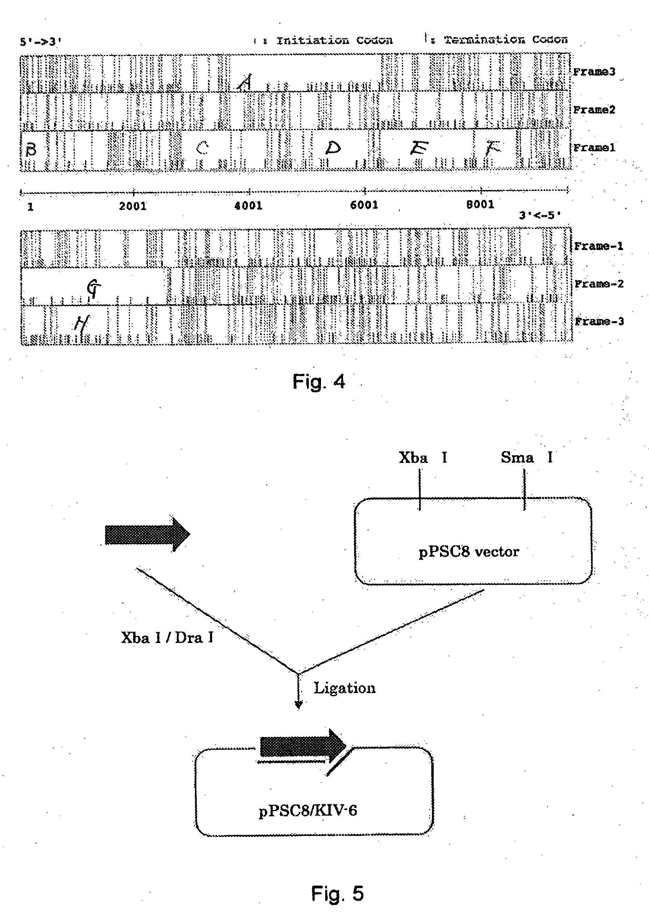

The nucleotide sequence determined as described above (nucleic acid sequence of the present invention) is shown in SEQ ID NO: 1. Fig. 4 shows a result of the screening for the open reading frame candidate on the basis of the nucleotide sequence. As a result, 8 open reading frame candidates were found. The nucleotide sequences thereof are shown in SEQ ID NOS: 2 to 9, and the amino acid sequences encoded by the nucleotide sequences are shown in SEQ ID NOS: 10 to 17.

### Example 2

### Method for detecting nucleic acid sequence of the present invention

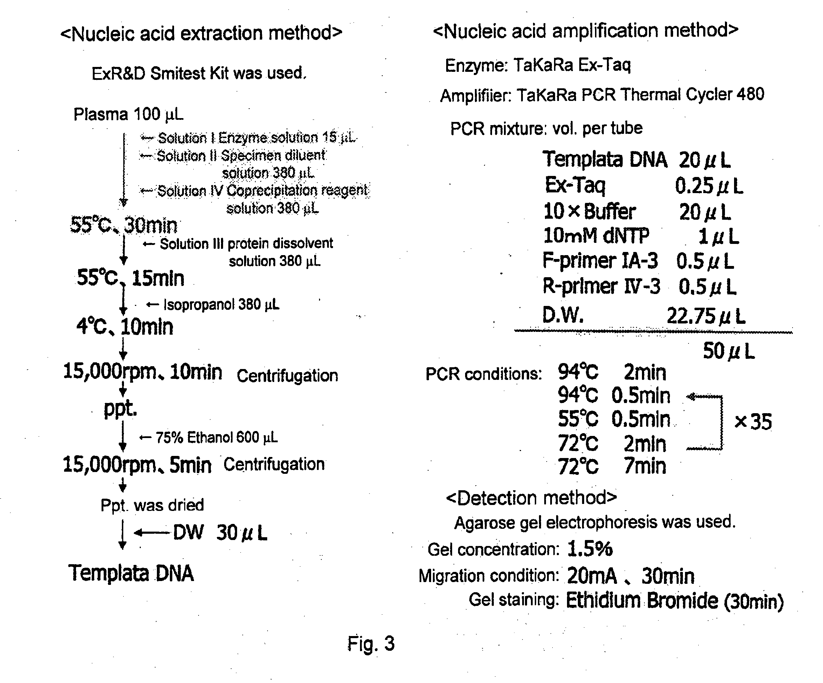

A plurality of polynucleotides were prepared on the basis of the nucleic acid sequence identified in Example 1 to investigate whether or not the polynucleotides were usable as the primer. Specifically, oligonucleotides of SEQ ID NOS: 18 to 39 were synthesized. The ability to amplify the nucleic acid sequence of the invention was tested for combinations of SEQ ID NOS: 18 and 19, 19 and 20, 22 and 23, 24 and 25, 26 and 27, 28 and 29, 30 and 31, 32 and 33, 34 and 35, 36 and 37, and 38 and 39. It was confirmed that they were usable equivalently. The test condition was as shown in Fig. 3. Therefore, it was confirmed that the nucleic acid sequence of the present invention was successfully detected by means of PCR based on the use of the primers.

### Example 3

### Presence of nucleic acid sequence of the present invention in clinical specimen including patient of hepatitis of unknown cause

If the nucleic acid sequence of the present invention originated from a pathogen of the hepatitis of unknown cause, it was expected that the nucleic acid sequence of the present invention would not be detected in normal humans, whereas it would be detected in a case of disease of hepatitis of unknown cause. In order to verify the clinical usefulness of the nucleic acid sequence of the invention, it was investigated whether or not the nucleic acid sequence of the present invention was present, by using the nucleic acid sequence detecting method established in Example 2 (primers to be used: SEQ ID NOS: 18 and 19 (1st PCR) and SEQ ID NOS: 20 and 21 (2nd PCR) in specimens from normal human, non-A, non-B, and non-C hepatitis patients, specimens of patients of known viral hepatitis (HBV and HCV), and specimens (plasmas) of patients of liver diseases, i.e., fatty liver, cholestatic hepatitis, primary biliary hepatitis, alcoholic hepatitis, and alcoholic cirrhosis. The results obtained are shown in the following table.

**Table 2**

| Detection rate of nucleic acid sequence of the invention in patients of non-A, non-B, and non-C hepatitises and patients of various liver diseases | | |
|---|---|---|
| Patient group | Positive number /total test number | Positive rate |
| Normal human | 0/14 | 0% |
| non-A, non-B, non-C hepatitis patients | 6/12 | 50% |
| Known viral hepatitis patients | 5/56 | 9% |
| Fatty liver | 1/7 | 15% |
| Cholestatic hepatitis | 1/4 | 25% |
| Primary biliary hepatitis | 14/24 | 58% |
| Alcoholic hepatitis | 1/11 | 9% |
| Alcoholic cirrhosis | 7/10 | 70% |

As shown in the results, the positive rate was extremely low in the normal humans and the known viral hepatitis patients. On the other hand, the positive rate was extremely high in the non-A, non-B, and non-C hepatitis patients, the primary biliary hepatitis, and the alcoholic cirrhosis. The causes of the diseases of the primary biliary hepatitis and the alcoholic cirrhosis have not been established yet. A possibility is suggested such that the pathogen, which has the novel nucleic acid of the present invention, may be the cause.

### Example 4

### Properties of nucleic acid sequence of the present invention

### (1) Possibility or impossibility of reverse transcription

A ExR&D Smitest Kit (Medical & Biological Laboratories Co., Ltd.) was used to extract the nucleic acid from the specimen. 15 µL of Solution I (enzyme solution), 380 µL of Solution II (specimen diluent solution), and 5 µL of Solution IV (coprecipitation reagent solution) of the kit were added to 100 µL of blood plasma, followed by incubation at 55°C for 30 minutes. Subsequently, 250 µL of Solution III (protein dissolvent solution) was added and incubated at 55°C for 15 minutes. Subsequently 600 µL of isopropanol was added and incubated an ice for 10 minutes. After the incubation, centrifugation was performed at 15,000 rpm for 10 minutes to obtain a precipitate to which 600 µL of 75% ethanol was added. A centrifugation was performed again at 15, 000 rpm for 10 minutes, and the obtained precipitate was dried. 30 µL of distilled water was added to the dried precipitate so that the precipitate was dissolved. After extracting the nucleic acid, DNase at a final concentration of 10 U and magnesium at a final concentration of 10 mM were added to perform a treatment at 37°C for 30 minutes. 50 µL of a mixture of phenol : chloroform (1 : 1) was added to the treatment solution, followed by being agitated and centrifuged at 15,000 rpm for 15 minutes. 1/10 volume of 8 M ammonium acetate and 2.5-fold volume of ethanol were added to the supernatant, followed by being stationarily placed at -80°C for 20 minutes. Centrifugation was performed at 15,000 rpm for 20 minutes, and the obtained precipitate was recovered as the nucleic acid. 20 µL of distilled water was added to the obtained nucleic acid. 2 µL of 0.1 M DTT, 1 µL of 10 mM dNTP, 1 µL of 40 U/µL of RNase (ribonuclease) inhibitor (Rnasin, PROMEGA), 1 µL of 200 U/µL of reverse transcriptase were added to 10 µL of the nucleic acid solution, followed by being reacted at 42°C for 50 minutes to synthesize cDNA. After further performing the reaction of 1st PCR, the presence of an amplification product was assessed by gel electrophoresis. As a result, no amplification product was detected. Therefore, it was judged that the nucleic acid in question was not RNA but DNA.

Further, after extracting the nucleic acid, RNase treatment was performed. 50 µL of a mixture of phenol : chloroform (1 : 1) was added to the reaction solution, followed by being agitated. A centrifugation was performed at 15,000 rpm for 15 minutes. 1/10 volume of 8 M ammonium acetate and 2.5-fold volume of ethanol were added to the obtained supernatant, followed by being stationarily placed at -80° for 20 minutes. A centrifugation was performed at 15,000 rpm for 20 minutes, and the precipitate was recovered as the nucleic acid. The reaction of 1st PCR was performed by using the obtained nucleic acid. As a result, an amplification product was detected. According to the results described above, it was revealed that the nucleic acid in question was DNA.

### (2) Demonstration of state of nucleic acid (to verify free state or state of being surrounded by capsid

4 µL of Cloned DNase 1 (TaKaRa) at a final concentration of 8 U and magnesium at a final concentration of 10 mM were added to 100 µL blood plasma, followed by being treated at 37°C for 30 minutes. A control was provided, which was prepared by treating extracted DNA with DNase 1, without providing blood plasma. The nucleic acid was extracted from the specimen after the treatment by using a ExR&D Smitest Kit (Medical & Biological Laboratories Co., Ltd.) as follows. 15 µL of Solution I (enzyme solution), 380 µL of Solution II (specimen diluent solution), and 5 µL of Solution IV (coprecipitation reagent solution) of the kit were added to 100 µL of blood plasma, followed by performing incubation at 55°C for 30 minutes. Subsequently, 250 µL of Solution III (protein dissolvent solution) was added and incubated at 55°C for 15 minutes, and then 600 µL of isopropanol was added and incubated at 4°C for 10 minutes. After the incubation, centrifugation was performed at 15,000 rpm for 10 minutes to obtain a precipitate to which 600 µL of 75% ethanol was added. A centrifugation was performed again at 15,000 rpm for 10 minutes, and the obtained precipitate was dried. 30 µL of distilled water was added to the dried precipitate so that the precipitate was dissolved. A 10-fold dilution series of the dissolved nucleic acid was prepared to measure the end titer. Primers of SEQ ID NOS: 18 and 19 were used under the condition of 1st PCR, and SEQ ID NOS: 20 and 21 were used for 2nd PCR. As a result, the specimen not treated with DNase and the specimen treated with DNase were detected while providing the same value of the end titer. No amplification was detected with a specimen in which the extracted DNA was treated with DNase in order to measure the function and effect of DNase. This confirmed that DNase functioned sufficiently. According to the results as described above, it was successfully demonstrated that DNA did not exist in a free state in blood plasma, but rather in a state of being surrounded by capsid.

### (3) Restriction enzyme reaction SalI digestion

A ExR&D Smitest Kit (Medical & Biological Laboratories Co., Ltd.) was used to extract the nucleic acid from the specimen. 15 µL of Solution I (enzyme solution), 380 µL of Solution II (specimen diluent solution), and 5 µL of Solution IV (coprecipitation reagent solution) of the kit were added to 100 µL of blood plasma, followed by incubation at 55°C for 30 minutes. Subsequently, 250 µL of Solution III (protein dissolvent solution) was added and incubated at 55°C for 15 minutes, and then 600 µL of isopropanol was added and incubated on ice for 10 minutes. After the incubation, a centrifugation was performed at 15,000 rpm for 10 minutes to obtain a precipitate to which 600 µL of 75% ethanol was added. A centrifugation was performed again at 15,000 rpm for 10 minutes, and the obtained precipitate was dried. 45 µL of distilled water was added, to the dried precipitate so that the precipitate was dissolved. After the dissolution, 5 µL of 10-fold concentration H buffer (buffer provided with restriction enzyme) (produced by TaKaRa) and 4 µL of *Sal*I (produced by TaKaRa) (20,000 U/ml) were added, followed by being mixed and reacted at 37°C for 90 minutes. 50 µL of phenol : chloroform (1 : 1) mixture was added to the reaction solution, followed by agitation. A centrifugation was performed at 15,000 rpm for 15 minutes. 1/10 volume of 8 M ammonium acetate and 2.5-fold volume of ethanol were added to the obtained supernatant, followed by being stationarily placed at -80°C for 20 minutes. A centrifugation was performed at 15,000 rpm for 20 minutes to recover the nucleic acid as a precipitate. DNA not subjected to the *Sal*I enzyme treatment was provided as a control. PCR was performed with primers interposing the restriction enzyme cleavage site. As a result, no amplification was detected for DNA digested with *Sal*I, but amplification was detected for DNA not digested with *Sal*I. According to this fact, it was successfully demonstrated that the objective DNA is cut by the restriction enzyme and the DNA is double stranded DNA.

### Example 5

### Screening for nucleotide sequence in clinical specimen

Screening for the nucleotide sequence was performed in relation to the clinical specimen which was PCR positive in Example 2. Long-PCR (LA-taq) was firstly performed for the extracted nucleic acid. The composition of the reaction solution included 0.5 µL of LA-taq, 25 µL of 2 x GC(1) buffer, 8 µL of 2.5 mM dNTP, 0.5 µL of the primer of SEQ ID NO: 22, 0.5 µL of the primer of SEQ ID NO: 39, and 16 µL of the extracted nucleic acid solution. PCR was performed under the following PCR conditions.

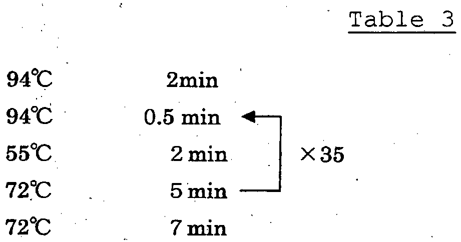

After the completion of LA-Taq PCR, PCR was performed again with the following combinations of the primers of SEQ ID NOS: 22 to 39 or the complementary sequences thereof. The condition shown in Fig. 3 was adopted as the condition to perform PCR. After the completion of PCR, agarose gel electrophoresis was performed, the band was cut out, and a sequencing reaction was performed to determine the nucleotide sequence. Sequence match of 98 to 100% was confirmed by making comparison with the nucleotide sequence of SEQ ID NO: 1 already determined.

**Table 4**

| Primer combination | |
|---|---|
| 1. | SEQ ID NOS: 22-23 |
| 2. | 23-24 |
| 3. | 2.4-25 |
| 4. | 25-26 |
| 5. | 26-27 |
| 6. | 27-28 |
| 7. | 29-30 |
| 8. | 30-31 |
| 9. | 31-32 |
| 10. | 32-33 |
| 11. | 33-34 |
| 12. | 34-35 |
| 13. | 35-36 |
| 14. | 36-37 |
| 15. | 37-38 |
| 16. | 38-39 |

### Example 6

### Expression of protein encoded by open reading frame (ORF)

### (1) Construction of expression vector

Target genes, which contained five ORF's (ORF's of No. 1: 3070 to 3669 bp, No. 2: 3826 to 4293 bp, No. 3: 5233 to 6117 bp, No. 4: 7198 to 7872 bp, No. 5: 7888 to 8610 bp) included in ORF's existing in the nucleotide sequence of SEQ ID NO: 1, were amplified by using primers shown in the following table, and 6 x His PCR products having XbaI, DraI restriction enzyme sites were obtained.

**Table 5**

| Expression primers | | | | |
|---|---|---|---|---|
| <Forward> | | | | |
| ORF | Primer name | F common (30mer) | Target | SEQ ID NO |
| 1-1 | 1-1F | TGATCTAGAATCGCATCATCATCATCATCAT- | TCTCGTGAACTCGTCTCGTCGTCATC | 43 |
| 1-2 | 1-2F | TGATCTAGAATGCATCATCATCATCATCAT- | GAATGGCATCTCCAACCCAAGATATC | 44 |
| 1-3 | 1-3F | TGATCTAGAATGCATCATCATCATCATCAT- | AAGAGGAAGTACAGGATCCATGTAGTG | 45 |
| 1-4 | 1-4F | TGATCTAGAATGCATCATCATCATCATCAT- | CGATCTGTGGTCGAGGTTTCATCTC | 46 |
| 1-5 | 1-5F | TGATCTAGAATGCATCATCATCATCATCAT- | AATAGGGAAATGATCTATACCCGCGT | 47 |
| | | Xba I HisHisHisHisHisHis | | |

| <Reverse> | | | | |
|---|---|---|---|---|
| ORF | Primer name | R common | Target origin | SEQ ID NO |
| 1-1 | 1-1R | TCATTTAAA- | TCAGAGTATGCTGCTGAGAGTTG | 48 |
| 1-2 | 1-2R | TCATTTAAA- | TCAATTGGGCTGGAGTGACTG | 49 |
| 1-3 | 1-3R | TCATTTAAA- | CTATTCGGAGGGGGGATGCGAT | 50 |
| 1-4 | 1-4R | TCATTTAAA- | TCACGGAAGATGGACGTACCTTG | 51 |
| 1-5 | 1-5R | TCATTTAAA- | CTAAAGCGGTCTACTCTGAAC | 52 |
| | | Dra I | | |

The 6 x His PCR product obtained as described above was subjected to the restriction enzyme treatment with XbaI/DraI. Subsequently, it was confirmed that the fragment having the desired length was obtained by means of the agarose gel electrophoresis. The fragment was mixed with pPSC8 (Nosan Corporation) enzyme-treated with XbaI/SmaI to perform the ligation reaction (Fig. 5). E. coli DH5α was transformed with the ligation product, and a clone, which had the sequence in question, was selected from transformants (pPSCB/KIV-6). The sequences obtained were analyzed, and the identical sequence was confirmed.

### (2) Cotransfection

200 µL of Sf900II (Invitrogen) including about 2 µg of expression vector DNA, 85 ng of Linear AcNPV DNA (Nosan Corporation), and 5 µL of Insect GeneJuice (Merck) was added to Sf9 cells (Nosan Corporation) of 1.0 x 10⁶ cells seeded in a 25 cm² flask. The cells were cultured at 28°C for about 6 days, and then a culture supernatant was recovered (cotransfection solution).

### (3) Simplified production of expression product

SF+ cells (Nosan Corporation), which were diluted with Sf900II to provide 1.5 x 10⁶ cells/mL, were prepared in an amount of 100 mL in a 250 mL Erlenmeyer flask. The cotransfection solution prepared in (2) was added thereto, and the cells were cultured with shaking at 130 rpm at 28°C. The cell culture solution was recovered after 72 hours, and centrifugation was performed at 3,000 x g at 4°C for 30 minutes separate a precipitate and a supernatant.

### (4) Purification of 6 x His binding expression protein

The precipitate and the supernatant, which were centrifuged and separated at 3,000 x g, were purified in accordance with the His Trap method by using His Trap Affinity Columns produced by NoVagen.

### Example 7

### Detection of antibody against expressed polypeptide

Any antibody contained in specimen was detected by means of EIA with a solid phase on which the protein purified in Example 6 was immobilized. The following were used as the specimen (blood plasma). General specimen: normal human specimen having ALT value of not more than 61 IU/L;
ALT abnormal human specimen: specimen suffered from hepatopathy having ALT value of not less than 61 IU/L.

EIA was carried out as follows.

### EIA (measurement of antibody)

Method: The sample was diluted in 0.2 M Carbonate Buffer pH 9.5 so that the protein amount was 5 µg/well, followed by being left to stand in 96-well plate at 4°C overnight to form a solid phase. After forming the solid phase (immobilization), washing was performed three times with a washing solution (23.8 g of Na₂HPO₄•12H₂O, 5.23 g of NaH₂PO₄•2H₂O, 42.5 g of NaCl, 2.5 ml of Tween 20 /L). 100 µL of 20% bovine serum / PBS was added to perform blocking for 60 minutes. After blocking, washing was performed three times, and 50 µL of the specimen, which was diluted 50 times with 20% bovine serum / PBS, was added to perform the reaction for 60 minutes. After the reaction, washing was performed five times, and antiHuman IgG Peroxidase-labeled antibody (Jackson Immuno Research), which was diluted 5000 times with 20% bovine serum / PBS, was added to perform the reaction for 60 minutes. After the reaction, washing was performed five times. The color was developed with OPD (for 30 minutes in a dark place), and then the measurement was performed at two wavelengths of 492 nm and 640 nm.

A suppression test was performed with the expressed protein in relation to the specimen which exhibited the positive result. The suppression test was performed in the same manner as the method for measuring the specimen, except that the specimen to be used was obtained by adding 50 µg of the expressed protein to the EIA positive specimen and performing the reaction for 60 minutes. Those specimens having the suppression rate of not less than 50% were designated as antibody positive.

The presence or absence of the nucleic acid sequence was also measured for the specimen described above by using the nucleic acid sequence-detecting method described in Example 2. The primers used were as follows, and the condition of PCR was as shown in Fig. 3.
1st PCR: SEQ ID NO: 18 primer 101-C
   : SEQ ID NO: 19 primer N101-B
2nd PCR: SEQ ID NO: 20 primer KS-2
   SEQ ID NO: 21 primer N101-D

The results are shown in the following table. In the table, PCR- indicates the PCR negative, and PCR+ indicates the PCR positive. According to the results, it is acknowledged that the antibody against the expressed protein exists in the ALT abnormal specimen.

**Table 6**

| Results: | | | | | | |
|---|---|---|---|---|---|---|
| Objective ORF | | No. 1 | No. 2 | No. 3 | No. 4 | No. 5 |
| General specimen | PCR- | 0/100 | 0/100 | 0/100 | 0/100 | 0/100 |
| | | | | | | |
| ALT abnormal specimen | PCR- | 18/100 | 10/100 | 20/100 | 22/100 | 5/100 |
| | PCR+ | 0/6 | 0/6 | 0/6 | 0/6 | 0/6 |

### Example 8

### Immunoprecipitation reaction of virus with EIA positive specimen

The immunoprecipitation reaction of virus was investigated by means of the following method, using three samples of positive specimens and three samples of negative specimens which exhibited the positive and negative results with respect to the expressed proteins Nos. 1, 2, 3, and 4 in Example 6 respectively.

100 µL of the PCR positive specimen and 100 µL of the EIA positive specimen or the EIA negative specimen diluted 50 times were mixed with each other, followed by being reacted for 60 minutes (each of the specimens was allowed to pass through a 0.2 µm filter as a pretreatment before the mixing and the centrifugation was performed at 15,000 rpm for 10 minutes). After the reaction, a centrifugation was performed at 12,000 rpm for 10 minutes. The supernatant was discarded and the pellet was washed three times with Saline (centrifugation at 12,000 rpm for 5 minutes). After the washing, 100 µL of Saline was added, and the nucleic acid was extracted to perform the PCR measurement.

The results are shown in the following table. According to the results, it is acknowledged that the antibody contained in the EIA positive specimen recognizes a virus particles.

**Table 7**

| Results of PCR measurement of respective immunoprecipitates | | | |
|---|---|---|---|
| | PCR | | |
| Only PCR positive specimen | - | | |
| PCR positive specimen and Saline | - | | |
| PCR positive specimen and EIA positive specimen | + | + | + |
| PCR positive specimen and EIA negative specimen | - | - | - |

### DESCRIPTION OF SEQUENCES

SEQ ID NO: 1: nucleotide sequence of KIV9496 (9496 nucleotides)
SEQ ID NO: 2: nucleotide sequence of deduced ORF-A
SEQ ID NO: 3: nucleotide sequence of deduced ORF-B
SEQ ID NO: 4: nucleotide sequence of deduced ORF-C
SEQ ID NO: 5: nucleotide sequence of deduced ORF-D
SEQ ID NO: 6: nucleotide sequence of deduced ORF-E
SEQ ID NO: 7: nucleotide sequence of deduced ORF-F
SEQ ID NO: 8: nucleotide sequence of deduced ORF-G
SEQ ID NO: 9: nucleotide sequence of deduced ORF-H
SEQ ID NO: 10: amino acid sequence of deduced ORF-A
SEQ ID NO: 11: amino acid sequence of deduced ORF-B
SEQ ID NO: 12: amino acid sequence of deduced ORF-C
SEQ ID NO: 13: amino acid sequence of deduced ORF-D
SEQ ID NO: 14: amino acid sequence of deduced ORF-E
SEQ ID NO: 15: amino acid sequence of deduced ORF-F
SEQ ID NO: 16: amino acid sequence of deduced ORF-G
SEQ ID NO: 17: amino acid sequence of deduced ORF-H
SEQ ID NO: 18: primer 101-C CAACACCgCAATCACAAAgT (3007-3026)
SEQ ID NO: 19: primer N101-B AACATTgAAACgTCATgTCC (3445-3464)
SEQ ID NO: 20: primer KS-2 CTCgTCTCgTCgTCATCgTA (3082-3101)
SEQ ID NO: 21: primer N101-D CATTTgCTCCCgCTggAgATg (3365-3385)
SEQ ID NO: 22: primer K-5 AACgATCCggACATCCACCACCA (145-167)
SEQ ID NO: 23: primer KIV-21 gCgATCgAggCATggCTCAA (536-555)
SEQ ID NO: 24: primer 101-1a CTgCAgAgCATCATgCggAg (796-815)
SEQ ID NO: 25: primer 101-T TCgATCCgCTTTCggTACgT (1383-1402)
SEQ ID NO: 26: primer X-2 ACCTAATgAAgACgCCgAAT (2290-2309)
SEQ ID NO: 27: primer KS-1 gAgACATggTgTAAgAgTCg (3057-3076)
SEQ ID NO: 28: primer 101-11 AgTggATgAAgCTggACCTT (3887-3906)
SEQ ID NO: 29: primer Op-3 gATCTTgATCCTgTACTCTT (4416-4435)
SEQ ID NO: 30: primer 101-21 gCgAAAgAggATTCTCgACT (4788-4807)
SEQ ID NO: 31: primer X-7 TgggAgTATggAgTCgACAT (5331-5350)
SEQ ID NO: 32: primer 101-23 TACACTAAAATCgACTCCTCC (6047-6067)
SEQ ID NO: 33: primer X-13 ATgAgTTTgAgAACgATCTT (6733-6752)
SEQ ID NO: 34: primer 101-29 ACCTgCgCCTgAggCTACgA (6888-6907)
SEQ ID NO: 35: primer X-17 gCAgATCgTTTCCgTTTggg (7564-7583)
SEQ ID NO: 36: primer 101-38 AACATCgAAAgATTAAAgAAA (7940-7960)
SEQ ID NO: 37: primer 101-6R CACgCgATTCCCATATCCCT (8789-8808)
SEQ ID NO: 38: primer KIV-14 CgTgTACTAACTATACTgAC (9255-9274)
SEQ ID NO: 39: primer KIV-16 CTCAAATTCCATCCgAATAg (9425-9444)
SEQ ID NO: 40: helicase gene detecting primer IA-3
SEQ ID NO: 41: helicase gene detecting primer IV-4
SEQ ID NO: 42: helicase gene detecting primer III R-2
SEQ ID NO: 43: primer 1-1F
SEQ ID NO: 44: primer 1-2F
SEQ ID NO: 45: primer 1-3F
SEQ ID NO: 46: primer 1-4F
SEQ ID NO: 47: primer 1-5F
SEQ ID NO: 48: primer 1-1R
SEQ ID NO: 49: primer 1-2R
SEQ ID NO: 50: primer 1-3R
SEQ ID NO: 51: primer 1-4R
SEQ ID NO: 52: primer 1-5R

<110> (Japanese Red Cross Society)
<120> Nucleic acid occuring in blood from hepatitis patient and polypeptide encoded by it
<130> C9180-PCT
<150> JP 2008-200050
   <151> 2008-08-01
<160> 52
<210> 1
   <211> 9496
   <212> DNA
   <213> Virus
<400> 1
   agtttggttt gagaggaaaa agaggggact cacgggaaca cgcttgacgg cttcaatgac      60
   gatactggat ccatcatgtc cgttccccca attcctagtg agaacgccgc atcggtcggt     120
   agcggtataa tggacgagtg ggggaacgat ccggacatcc accaccagtg tatcgccatc      180
   gttatccgtg gacggtccct gtccacccca catgatccag ccggcatgga ccaggacaag     240
   ccccaggtca gagtcatcgg tataaacatc cgtaccccag atccccctct tctcccatcc     300
   ggcactaatc tccccacgcc ctttgccaac accttcacgg atcttgaccg gacacgagga     360
   tgcatcttcc catccaggtg ctcctctcaa tttccagttc ttcccaagga accccccacc     420
   tatgtggacc ttgacgagtc caccctgtag atttcgagaa cggtgacgag ataaacgata     480
   atccgaaccg gtatacgtct cggtagctac aacccgattc atttccgatt tcgtcttgag    540
   ccatgcctcg atcgcggtac tcttgacatc gggatattta cgtgtggcct cggtcaaatg     600
   actgaagtcg cgtattcgaa cgggtgattt aggtcggtcc tcgtctttac gccttttcga    660
   cttgttggca tttcctcgcc tagcatcgtc gacggcagca tccattcgag gtcgtttatc     720
   ccttggagtg tattgataat cccttgaatc cgtcttgtat tcatacggac gtggttctcg     780
   cctaggggcg acttgctgca gagcatcatg cggagtctgc gctgcaggat gggcaggatt     840
   accgtcgagg tatcgcctga attgatcccg attcgcttga tccatacgtt ggtttgcttc     900
   tttgatacgt gctgaattga tctcggcttg tcgagcatat gccagtcgtt cctgctcctt     960
   ttgcctctct ttagcttccc tagcttctcg gacttccttt tccctctgag cagccgcagc    1020
   acgcacacga gccatacgag cttcttgttc atcctgaagt cgtttcctct ctcgatcgaa    1080
   tcgatcttgc tctgccgtcg tatgtgctct ttgagcagca tggctagccg agttaccggt    1140
   ccatccacca aacgatccat actcgaatcc tccactgaat ggcatgaacc cgccgtatcc    1200
   gggatagttg gaaccgggga aatctcgtcc aggtggtcgt gctgctgacg aggtggcagc    1260
   tgttgcgggt ttgggagcgg cagaggcggc agaagcggat cgagggggtt cacgagccga   1320
   tgggagggga ggttgggagg tgttgggcgt cgacttggca tcacgagctc ctccaaaggc     1380
   ggacgtaccg aaagcggatc gagcgccagg ttgagatgtt gcggatttgc ctggactgag    1440
   ctggccgggg cccaaagcag caggatcggg gcgtgttcca gtccctccac tgggcgtgcg    1500
   acggtgagct acaccaggag cttgtggagc cgttgctgat ggtggacgtt ctcgttgggg    1560
   atcgaatggt gagggtaagg atcggccgcc gatggcagcg agtcctggta ggggtagatt    1620
   cgggcgctgg cttgagccgg tagcggttgc tgttggaggt agagcggggg agggtttgtt    1680
   gggaatcact ggggatgccg cggatggtaa agagcgggca ttcgtaggcg acggtggaag    1740
   tgatggacga gggtatgggt ttgccggtgt cccagtggat gcgcgtggtg atgtcgctgg    1800
   gttcgccttg gccgctctat cctttctctc ctgctcaatc ctcgcctgtt ccctctctcg    1860
   ctcacgctga cgagcccttt cacgttccct cagatcttgc tctgataatc ccgacccacc    1920
   aaacatactt cgatagccgt aattgaatgg ggcttcgcgc ttgggaagtg atgcgctggc    1980
   agtcgccggt gtcgtggaag ttgcggcggt agcaggagtt gaggctggtg tcggtgccgt    2040
   tgtaccatga gggtggtgat ggtgatggtg gtgatggtgg tgaggtgctc ctgggtgtga    2100
   atgctggatc gtaggagagg cggtagccgg tgtagcaggt tttggttcgt gacgcgtgct    2160
   tccgatcgta tatttgggaa cgtctgccgt cagctccgac atctcgccta tagttaactc    2220
   accagcgact ggaggtggat ctctcgatcc aaaaggacta taactaggtc tcaatccatt    2280
   gccgggacca cctaatgaag acgccgaatt gttccccggt ctagctccgg tatcgccgag    2340
   tcctgcacct gtcaggctgg ggctactgct tgctgggttc ctgggtcgtt ggaggcctcc    2400
   aaaattggag aatgatctga tgatggtggc ttcttcggcg cgaggtggtc ccgaggtctg    2460
   agggaggtta gtgcccggtc gttgggggag tgggtcggtt ggtttcggct gggggtttgt   2520
   cgaggcgtgg ggagaaggca tcgtggttgg gcgttccggt ggatcgtatg tctagttgta    2580
   ttatatgtcg ccaagagatg agagtaggta ttatgctatg tgaacatgtg atgtatgaca    2640
   ataaggatat gcgaattgat ggttgaattg aaaggatgaa gatgacacag tttggcgttg    2700
   caataagcgt agaagttggt ccctgaaccc tgaaagtgtg tggacttgtg tatgccctag   2760
   attacccctg tgcctgagta gcagaatcct ggtaaacccg tttaaccgaa tgtggcactt   2820
   ttaataggcg tgactgcttt ctttcattca acctccactg tcaacaaaca actcagctat   2880
   acactcatac tcatacactc cttcgaccat acatattgct atacccacac tcgttctgcc   2940
   tgccttacct ggctgctaag tcaacaactc gaccacgacg cgtatcaacc atctcgaaac   3000
   acccgccaac accgcaatca caaagtaaac aatagatccg aaacgtcaca ttctaccgac   3060
   tcttacacca tgtctcgtga actcgtctcg tcgtcatcgt atccactttc caatcccacc   3120
   ccatcttcca gcatgaaatc cagccagtca cgtataccgg tgaaagccaa gagagcacca   3180
   ctaggcgaaa gagtcgacaa ccacgatcac accactcccc gtcagcacct cgtcaagtct   3240
   gtcaaatcag tcatcagacc tcggatcatc tctacaaagt cgaccgctag tccatcaaag   3300
   tcgtccacat accgaccatc acctcgagcg gcagtacaag gttccccttc atcctcaatc   3360
   gtttcatctc cagcgggagc aaatgtctcg agacccggac atacacccca ccctcgccgc   3420
   tcatctgttg gtctcgcatt ccttggacat gacgtttcaa tgttgatgga tactactcgg   3480
   ctggattttg tgagcgatac ggatgatagt gagggagatg agccattgga caatatgctc   3540
   gtgaggaggg gagtgatggg accagggaga ttgatgacgc cagccaatag tcaggaatct   3600
   caggtaagct cattgtgcct cttggagtct cagctgacat gtagcccaac tctcagcagc   3660
   atactctgaa ggagccatct ccacgtcgtt tggctatacg attacctaca cctccagcat   3720
   ctcaatcagg taccgatgtc gaggaaacca tctctcgaac gcttgtccga ccgagaccag   3780
   cacctacacc tccacgtcga tctgctggcc cttcgatcct gctgaatgga atggcatctc   3840
   caacccaaga tatctcaccc aatccccgaa gaaaaaagtc tcccagagtg gatgaagctg   3900
   gaccttcgca acttcgatcc ccattcgaga ttgcccctac tactacttcg cctcgtcatc   3960
   ccagggatag agtcgagatt gtcattggcc agccatcgcg taaatcagtc tcgccctcgg   4020
   ccacgcccaa agctagcaaa ccaaggaaga gcagagtatc cactaaaacc ccggcttctc   4080
   acccattatc acaatcaacc tcttcccaac cctctcgagc gaaaagggca ccggcaacag   4140
   cccctcaacc gaatagacga aagtcaacac cagccactcg taaaccctct ccacctagaa   4200
   cgacagctaa acccaaattc gctaaaacac ccaagcggct ctcactccca ggtcgaggtc   4260
   gtgctcggaa atcagtcact ccagccgaat tgaaagcgat gattggttta ctgccggagc   4320
   cagtggagca gagtagtccc tccgacgatc ccctgctttt agttagcgac gacgaggacc   4380
   acacacccgc tatcagacct tcatctcatt ctgggaagag tacaggatca agatcggctg   4440
   ctaggattat cgatttcgat gctgccattc aaggtcgctc gagcgtagag gaggtcgtga   4500
   gggattacga ggtacctatc cacgggtcgg aaccggatta tgatgtcgct cgggattatg   4560
   gatttgattc aggtgattca gatgaggaaa tggggaatga tacatttgtg aatgtcgccg   4620
   gagctcgagc gaaggtgaat ccggcggatc atcaggacga ggcaacccta gaagatgagg   4680
   atgagaatgg aagtcacctc agctcaagat caaactcgcc attcgcattt caagaagcgg   4740
   aagtacgatt ctcttcacct gctctttcgc tccgagccgc cagcccagcg aaagaggatt   4800
   ctcgactcgt ctcttcgccc gtcatcgcct ctgtcgcctc gcctgttcca tcagtcgccg   4860
   ctagtctacc ctcgcctttc ctctcacgct cgacgaactc gcctcgattg agcagatcaa   4920
   ccactgctac gccggtcgtt cattcccatg gcctaccatc gcctgctcct tcctcgcctc   4980
   cgcccacgga gatggagcca gaggtggcat cctcgcctcg ttatgcccgg tcccagacag   5040
   gtatccctcg agctacagtg gctgtaaacc aggagctcga gtccgctacc gtaccagctt   5100
   ctcccgccta cagcgaaaag ttctctgttg gtatgctctc agctccctcg tctccgttct   5160
   ctaccagatc acgcgataaa tccgccacgc ctcgattcac agaacaatta tcagtacatt   5220
   cgcagggaat ggatgaagag gaagtacagg atccatgtag tgctagtcca ctactcctac   5280
   ctggaacacc atccgccctt ggtctcttta tgcctgaaga agcggatggg atgtcgactc   5340
   catactccca cattggggcg gagcctgata ctcctgcccc aaactttgat tcgccctcac   5400
   ggtcaagcat acatatggac tcgccagctc cagtcgaagc tcggacatcg ccaactctct   5460
   ttggttcacg ggtcgcatcg ccagcccgct ccgttgttgc tcaagtcgct tcgcccgtca   5520
   gacctatggc tactcaggtc gcatcacctg ttcgttcttc tgcttctcag atggattcgc   5580
   ctgctcgctc tgtttctcga gctggatcgc ctgccctttc ttctgcttct cgaattggat   5640
   cgcctgccag accagtcact ctacaggctg catcgcctgc tcgttccact acttcccagg   5700
   cagctgagct gtccatgggc tattctcaat caccgagaag atctccggtc tgtctacctc   5760
   aagagatgga tctacccgaa gaaataccga ccgtcagcac cagctcgccc aggaggtccc   5820
   cggtctgcct cccttctcat gtggatgttg tggacaacgt cgaggagcag ccattaccag   5880
   tcgtcaacac ccattcgttt aggaagtcgc cactcgccta cgcccaatca cctcgaaggt   5940
   ctccagtctg cttacccgcc gacatggaga atgccgacga agatgaagct ggggataaag   6000
   agaacatggc gcctgttcaa tcgactactc caatcttatc accccctaca ctaaaatcga   6060
   ctcctccaat gtacgctttc actttctctc cccgaatcgc atcccccctc cgaatagccc   6120
   ataatcaggc atccgctctg aaacgagaca gcatcgctcg aacctctcaa tcacctatac   6180
   cagctgttga atcattccat gagagatttg cagaggttga tgaatgtccc agtgcggatg   6240
   tgacattaga ggggaacagt gcggattgga gtttgtagtg atgagggttt accggaggtg   6300
   gatcgtgagg aacatggact tggcttggac ctacaggggg aagatcaaga gaacgacgat   6360
   aacctgtcta tatccttatc gattgctgaa gaaggtgaag aggaggagga gagtgatctc   6420
   gtcgattctt ccatcgagca ggaagatgat ctggagcttg gagccgatcc cacgacccag   6480
   ctagcggacg acattgtcga ctcgaatgag gacgagcaag atgctgaaga agatgcggag   6540
   ggatctgaag aggaggaggg gcaggaatcg aatgtgattg cgccaatccc gactttcacg   6600
   gacgaacgaa atgcagcaga ggaaggggag agtacggatt ctgaagaagc cccatccagc 6660
   gacgacgagc aggaggacga gaccgctcag gaagaattgg aggaggatat ccctgcttca 6720
   cctgcacctg tcaagatcgt tctcaaactc atcaatcggg gtattgtcaa gatcgaaccg 6780
   cctgcctctc ctgcccgggc caatgaagcg gacgcctctg aactccgatc gaccactccg 6840
   gcttatgatc cacctcagcc tatcgtcgcc gctcctatga ctcgatcacc tgcgcctgag 6900
   gctacgacag tgtaccccgc cctacctact acacccatca ccgcgcctct ctcgcctctt 6960
   gtgtccacca ctcccctcgg aactcctcca gccaagcaac gatcgcctgc cctgcagcag 7020
   agtctacccc aatcttccga ctacagcact ctgcaagctg gcccctctac tcgaccacga 7080
   tcccgactct ctcgtcagat tcccgccagt cccgaacaag aggaaagtga ttccatcaga 7140
   gtccgaactc ctcgtagtct cggtcttgag cttgaggatg ccgaggccga taatagtatg 7200
   cgatctgtgg tcgaggtttc atctcttgat cccaaagctg ccgctcgagc tgctgctatc 7260
   ttgaaacttg taagcttttt tgattctttg catggccaag ctgatatgca gaaccacgca 7320
   tacattgaac atggcgatat ttcgaaactt ggcgaatcga ctgctgctcg acatcaatcc 7380
   atgcactcgg ctcgactcga caagctcgat gcgtcccgtc gcgaagacaa gactgagctc 7440
   ttgtacgaag ccgagctgga aattgtttcg actcgccgaa gtcgatctcg gtccatgtct 7500
   cggttcagag gaatcagcga ggcacccact gaattgccat tgccaggcgc atggaatggt 7560
   actcccaaac ggaaacgatc tgctcatcta caaattgaag ctcctaagac aagcaacact 7620
   tggggtgtat ctcaatggaa gaaacttgaa aaggtcttta gggccgagag ggagatttgg 7680
   gtaacggagc gtaatgtcaa agcgatgcct ggcgggttta ttggttgggc aaggatgtct 7740
   acttttggac cgcctgctgc ggttgctgtc ccgtgggatc cgactcgggt ggtggatagg 7800
   ttcctggagg aacagggtat ctcgaaaaag gagcaagtgg gagattggtc aaggtacgtc 7860
   catcttccgt gatcttgaag ggagctgatg aatagggaaa tgatctatac ccgcgtcgaa 7920
   gctctcgaga ggcatgcgga acatcgaaag attaaagaaa cgtctcaaga agagagatca 7980
   cccaagaaac caaagactgc gcctgctcct ccgtcgaccg ttcggaagtt gtttggatgg 8040
   gtcatgccct ccgctaccaa gtctgctctt cccgtcgctg ccccagaaag cagtgtaaag 8100
   aatatggaca agggcaaagg gaaagagagg gagggtcgac agacattcaa ggatcgactt 8160
   gaggctcttc aggatcaacc catgactcag ctttcagagt cggtctcgat ccctcgacct 8220
   acgcccatcg ccagctcgac tcccaatacg gcaccacctc atccttcttc tcctgcttgg 8280
   acgaatgctt accagcccat ctcaaccgcc aacgcccacg ctagcggcag cactaccact 8340
   cgcccaactc cccgaacttt atctgcgatc ctagctgaat cgtcgtccac cacctccagc 8400
   cgaggacaac tccattcatc cttatctcaa cgatctgcgg ctctcgatgc gctgttcggt 8460
   ggatctacct cgacatcaac caggccggtc gtacagatca aaagaagttc gagcgttaaa 8520
   gatatagtca ggggatttga agattcgggg gctttggcta aaagtaccgg tgaggaaggg 8580
   ctgaggaggg ttcagagtag accgctttag aggttttgaa taacgaatta taacgaattt 8640
   tccttggatg tctttaatgt aaagtaataa tataatgcat atgtgcttgt ctgcataact 8700
   tatttgatga tcgatctgcg gacatgccaa gccattccaa ggcgccggtc gaccgaacct 8760
   tcctctcctc atccgccggt acatattcag ggatatggga atcgcgtgat ttgggacgat 8820
   atgaccacat cgataaccaa gctccactaa ctatcaaacc actgggtaaa gtatgcagag 8880
   ttgtctctga ttctgactta ttgttgtact gacgatggct caacctgttg atgttgaagt 8940
   aggtaacaaa gtggcggctc cggtagagag ggagaaggat gaggggacta ttcggactac 9000
   taggtacttt ccatctaatc ccatgacgag ggactgatga gagtgatcag ttgtcagaga 9060
   cgtatatgag gcaacagact ggagacgatg atgttcttgt caagaaggta tgtccctctt 9120
   attctgagtg aagagtggac acagtggggg cagaaagaga gctgagggca gatggtacat 9180
   gttaaccggg caatcaatga gattggatgg acgaaatacc atctcaagct attctgtgag 9240
   taccctcgtc ctttcgtgta ctaactatac tgacatccag ttttgtaagc tgtaacgccc 9300
   tctcgctgat ggaactgatg ggacaggaat ggattcgggt acgcagtgga tagtatgcta 9360
   ggtaagccca ctggctcgga tgaagctgat gaggtagttg tatgccaatc gattgctcaa 9420
   cctgctattc ggatggaatt tgaggggagg ggcgttacgc aattattcgc tgtatcactc 9480
   tcttctgctg ttggtc 9496
<210> 2
   <211> 2631
   <212> DNA
   <213> Virus
<400> 2
   cccaactctc agcagcatac tctgaaggag ccatctccac gtcgtttggc tatacgatta 60
   cctacacctc cagcatctca atcaggtacc gatgtcgagg aaaccatctc tcgaacgctt 120
   gtccgaccga gaccagcacc tacacctcca cgtcgatctg ctggcccttc gatcctgctg 180
   aatggaatgg catctccaac ccaagatatc tcacccaatc cccgaagaaa aaagtctccc 240
   agagtggatg aagctggacc ttcgcaactt cgatccccat tcgagattgc ccctactact 300
   acttcgcctc gtcatcccag ggatagagtc gagattgtca ttggccagcc atcgcgtaaa 360
   tcagtctcgc cctcggccac gcccaaagct agcaaaccaa ggaagagcag agtatccact 420
   aaaaccccgg cttctcaccc attatcacaa tcaacctctt cccaaccctc tcgagcgaaa 480
   agggcaccgg caacagcccc tcaaccgaat agacgaaagt caacaccagc cactcgtaaa 540
   ccctctccac ctagaacgac agctaaaccc aaattcgcta aaacacccaa gcggctctca 600
   ctcccaggtc gaggtcgtgc tcggaaatca gtcactccag ccgaattgaa agcgatgatt 660
   ggtttactgc cggagccagt ggagcagagt agtccctccg acgatcccct gcttttagtt 720
   agcgacgacg aggaccacac acccgctatc agaccttcat ctcattctgg gaagagtaca 780
   ggatcaagat cggctgctag gattatcgat ttcgatgctg ccattcaagg tcgctcgagc 840
   gtagaggagg tcgtgaggga ttacgaggta cctatccacg ggtcggaacc ggattatgat 900
   gtcgctcggg attatggatt tgattcaggt gattcagatg aggaaatggg gaatgataca 960
   tttgtgaatg tcgccggagc tcgagcgaag gtgaatccgg cggatcatca ggacgaggca 1020
   accctagaag atgaggatga gaatggaagt cacctcagct caagatcaaa ctcgccattc 1080
   gcatttcaag aagcggaagt acgattctct tcacctgctc tttcgctccg agccgccagc 1140
   ccagcgaaag aggattctcg actcgtctct tcgcccgtca tcgcctctgt cgcctcgcct 1200
   gttccatcag tcgccgctag tctaccctcg cctttcctct cacgctcgac gaactcgcct 1260
   cgattgagca gatcaaccac tgctacgccg gtcgttcatt cccatggcct accatcgcct 1320
   gctccttcct cgcctccgcc cacggagatg gagccagagg tggcatcctc gcctcgttat 1380
   gcccggtccc agacaggtat ccctcgagct acagtggctg taaaccagga gctcgagtcc 1440
   gctaccgtac cagcttctcc cgcctacagc gaaaagttct ctgttggtat gctctcagct 1500
   ccctcgtctc cgttctctac cagatcacgc gataaatccg ccacgcctcg attcacagaa 1560
   caattatcag tacattcgca gggaatggat gaagaggaag tacaggatcc atgtagtgct 1620
   agtccactac tcctacctgg aacaccatcc gcccttggtc tctttatgcc tgaagaagcg 1680
   gatgggatgt cgactccata ctcccacatt ggggcggagc ctgatactcc tgccccaaac 1740
   tttgattcgc cctcacggtc aagcatacat atggactcgc cagctccagt cgaagctcgg 1800
   acatcgccaa ctctctttgg ttcacgggtc gcatcgccag cccgctccgt tgttgctcaa 1860
   gtcgcttcgc ccgtcagacc tatggctact caggtcgcat cacctgttcg ttcttctgct 1920
   tctcagatgg attcgcctgc tcgctctgtt tctcgagctg gatcgcctgc cctttcttct 1980
   gcttctcgaa ttggatcgcc tgccagacca gtcactctac aggctgcatc gcctgctcgt 2040
   tccactactt cccaggcagc tgagctgtcc atgggctatt ctcaatcacc gagaagatct 2100
   ccggtctgtc tacctcaaga gatggatcta cccgaagaaa taccgaccgt cagcaccagc 2160
   tcgcccagga ggtccccggt ctgcctccct tctcatgtgg atgttgtgga caacgtcgag 2220
   gagcagccat taccagtcgt caacacccat tcgtttagga agtcgccact cgcctacgcc 2280
   caatcacctc gaaggtctcc agtctgctta cccgccgaca tggagaatgc cgacgaagat 2340
   gaagctgggg ataaagagaa catggcgcct gttcaatcga ctactccaat cttatcaccc 2400
   cctacactaa aatcgactcc tccaatgtac gctttcactt tctctccccg aatcgcatcc 2460
   cccctccgaa tagcccataa tcaggcatcc gctctgaaac gagacagcat cgctcgaacc 2520
   tctcaatcac ctataccagc tgttgaatca ttccatgaga gatttgcaga ggttgatgaa 2580
   tgtcccagtg cggatgtgac attagagggg aacagtgcgg attggagttt g 2631
<210> 3
   <211> 435
   <212> DNA
   <213> Virus
<400> 3
   gaggaaaaag aggggactca cgggaacacg cttgacggct tcaatgacga tactggatcc 60
   atcatgtccg ttcccccaat tcctagtgag aacgccgcat cggtcggtag cggtataatg 120
   gacgagtggg ggaacgatcc ggacatccac caccagtgta tcgccatcgt tatccgtgga 180
   cggtccctgt ccaccccaca tgatccagcc ggcatggacc aggacaagcc ccaggtcaga 240
   gtcatcggta taaacatccg taccccagat ccccctcttc tcccatccgg cactaatctc 300
   cccacgccct ttgccaacac cttcacggat cttgaccgga cacgaggatg catcttccca 360
   tccaggtgct cctctcaatt tccagttctt cccaaggaac cccccaccta tgtggacctt 420
   gacgagtcca ccctg 435
<210> 4
   <211> 861
   <212> DNA
   <213> Virus
<400> 4
   ccgaatgtgg cacttttaat aggcgtgact gctttctttc attcaacctc cactgtcaac 60
   aaacaactca gctatacact catactcata cactccttcg accatacata ttgctatacc 120
   cacactcgtt ctgcctgcct tacctggctg ctaagtcaac aactcgacca cgacgcgtat 180
   caaccatctc gaaacacccg ccaacaccgc aatcacaaag taaacaatag atccgaaacg 240
   tcacattcta ccgactctta caccatgtct cgtgaactcg tctcgtcgtc atcgtatcca 300
   ctttccaatc ccaccccatc ttccagcatg aaatccagcc agtcacgtat accggtgaaa 360
   gccaagagag caccactagg cgaaagagtc gacaaccacg atcacaccac tccccgtcag 420
   cacctcgtca agtctgtcaa atcagtcatc agacctcgga tcatctctac aaagtcgacc 480
   gctagtccat caaagtcgtc cacataccga ccatcacctc gagcggcagt acaaggttcc 540
   ccttcatcct caatcgtttc atctccagcg ggagcaaatg tctcgagacc cggacataca 600
   ccccaccctc gccgctcatc tgttggtctc gcattccttg gacatgacgt ttcaatgttg 660
   atggatacta ctcggctgga ttttgtgagc gatacggatg atagtgaggg agatgagcca 720
   ttggacaata tgctcgtgag gaggggagtg atgggaccag ggagattgat gacgccagcc 780
   aatagtcagg aatctcaggt aagctcattg tgcctcttgg agtctcagct gacatgtagc 840
   ccaactctca gcagcatact c 861
<210> 5
   <211> 984
   <212> DNA
   <213> Virus
<400> 5
   accaggagct cgagtccgct accgtaccag cttctcccgc ctacagcgaa aagttctctg 60
   ttggtatgct ctcagctccc tcgtctccgt tctctaccag atcacgcgat aaatccgcca 120
   cgcctcgatt cacagaacaa ttatcagtac attcgcaggg aatggatgaa gaggaagtac 180
   aggatccatg tagtgctagt ccactactcc tacctggaac accatccgcc cttggtctct 240
   ttatgcctga agaagcggat gggatgtcga ctccatactc ccacattggg gcggagcctg 300
   atactcctgc cccaaacttt gattcgccct cacggtcaag catacatatg gactcgccag 360
   ctccagtcga agctcggaca tcgccaactc tctttggttc acgggtcgca tcgccagccc 420
   gctccgttgt tgctcaagtc gcttcgcccg tcagacctat ggctactcag gtcgcatcac 480
   ctgttcgttc ttctgcttct cagatggatt cgcctgctcg ctctgtttct cgagctggat 540
   cgcctgccct ttcttctgct tctcgaattg gatcgcctgc cagaccagtc actctacagg 600
   ctgcatcgcc tgctcgttcc actacttccc aggcagctga gctgtccatg ggctattctc 660
   aatcaccgag aagatctccg gtctgtctac ctcaagagat ggatctaccc gaagaaatac 720
   cgaccgtcag caccagctcg cccaggaggt ccccggtctg cctcccttct catgtggatg 780
   ttgtggacaa cgtcgaggag cagccattac cagtcgtcaa cacccattcg tttaggaagt 840
   cgccactcgc ctacgcccaa tcacctcgaa ggtctccagt ctgcttaccc gccgacatgg 900
   agaatgccga cgaagatgaa gctggggata aagagaacat ggcgcctgtt caatcgacta 960
   ctccaatctt atcaccccct acac 984
<210> 6
   <211> 1620
   <212> DNA
   <213> Virus
<400> 6
   aggggaacag tgcggattgg agtttgtagt gatgagggtt taccggaggt ggatcgtgag      60
   gaacatggac ttggcttgga cctacagggg gaagatcaag agaacgacga taacctgtct     120
   atatccttat cgattgctga agaaggtgaa gaggaggagg agagtgatct cgtcgattct     180
   tccatcgagc aggaagatga tctggagctt ggagccgatc ccacgaccca gctagcggac     240
   gacattgtcg actcgaatga ggacgagcaa gatgctgaag aagatgcgga gggatctgaa     300
   gaggaggagg ggcaggaatc gaatgtgatt gcgccaatcc cgactttcac ggacgaacga     360
   aatgcagcag aggaagggga gagtacggat tctgaagaag ccccatccag cgacgacgag     420
   caggaggacg agaccgctca ggaagaattg gaggaggata tccctgcttc acctgcacct     480
   gtcaagatcg ttctcaaact catcaatcgg ggtattgtca agatcgaacc gcctgcctct     540
   cctgcccggg ccaatgaagc ggacgcctct gaactccgat cgaccactcc ggcttatgat     600
   ccacctcagc ctatcgtcgc cgctcctatg actcgatcac ctgcgcctga ggctacgaca     660
   gtgtaccccg ccctacctac tacacccatc accgcgcctc tctcgcctct tgtgtccacc     720
   actcccctcg gaactcctcc agccaagcaa cgatcgcctg ccctgcagca gagtctaccc     780
   caatcttccg actacagcac tctgcaagct ggcccctcta ctcgaccacg atcccgactc     840
   tctcgtcaga ttcccgccag tcccgaacaa gaggaaagtg attccatcag agtccgaact     900
   cctcgtagtc tcggtcttga gcttgaggat gccgaggccg ataatagtat gcgatctgtg     960
   gtcgaggttt catctcttga tcccaaagct gccgctcgag ctgctgctat cttgaaactt    1020
   gtaagctttt ttgattcttt gcatggccaa gctgatatgc agaaccacgc atacattgaa    1080
   catggcgata tttcgaaact tggcgaatcg actgctgctc gacatcaatc catgcactcg    1140
   gctcgactcg acaagctcga tgcgtcccgt cgcgaagaca agactgagct cttgtacgaa    1200
   gccgagctgg aaattgtttc gactcgccga agtcgatctc ggtccatgtc tcggttcaga    1260
   ggaatcagcg aggcacccac tgaattgcca ttgccaggcg catggaatgg tactcccaaa    1320
   cggaaacgat ctgctcatct acaaattgaa gctcctaaga caagcaacac ttggggtgta    1380
   tctcaatgga agaaacttga aaaggtcttt agggccgaga gggagatttg ggtaacggag    1440
   cgtaatgtca aagcgatgcc tggcgggttt attggttggg caaggatgtc tacttttgga    1500
   ccgcctgctg cggttgctgt cccgtgggat ccgactcggg tggtggatag gttcctggag    1560
   gaacagggta tctcgaaaaa ggagcaagtg ggagattggt caaggtacgt ccatcttccg    1620
<210> 7
   <211> 729
   <212> DNA
   <213> Virus
<400> 7
   agggagctga tgaataggga aatgatctat acccgcgtcg aagctctcga gaggcatgcg      60
   gaacatcgaa agattaaaga aacgtctcaa gaagagagat cacccaagaa accaaagact     120
   gcgcctgctc ctccgtcgac cgttcggaag ttgtttggat gggtcatgcc ctccgctacc     180
   aagtctgctc ttcccgtcgc tgccccagaa agcagtgtaa agaatatgga caagggcaaa     240
   gggaaagaga gggagggtcg acagacattc aaggatcgac ttgaggctct tcaggatcaa     300
   cccatgactc agctttcaga gtcggtctcg atccctcgac ctacgcccat cgccagctcg     360
   actcccaata cggcaccacc tcatccttct tctcctgctt ggacgaatgc ttaccagccc     420
   atctcaaccg ccaacgccca cgctagcggc agcactacca ctcgcccaac tccccgaact     480
   ttatctgcga tcctagctga atcgtcgtcc accacctcca gccgaggaca actccattca     540
   tccttatctc aacgatctgc ggctctcgat gcgctgttcg gtggatctac ctcgacatca     600
   accaggccgg tcgtacagat caaaagaagt tcgagcgtta aagatatagt caggggattt     660
   gaagattcgg gggctttggc taaaagtacc ggtgaggaag ggctgaggag ggttcagagt     720
   agaccgctt                                                            729
<210> 8
   <211> 2571
   <212> DNA
   <213> Virus
<400> 8
   acatacgatc caccggaacg cccaaccacg atgccttctc cccacgcctc gacaaacccc 60
   cagccgaaac caaccgaccc actcccccaa cgaccgggca ctaacctccc tcagacctcg 120
   ggaccacctc gcgccgaaga agccaccatc atcagatcat tctccaattt tggaggcctc 180
   caacgaccca ggaacccagc aagcagtagc cccagcctga caggtgcagg actcggcgat 240
   accggagcta gaccggggaa caattcggcg tcttcattag gtggtcccgg caatggattg 300
   agacctagtt atagtccttt tggatcgaga gatccacctc cagtcgctgg tgagttaact 360
   ataggcgaga tgtcggagct gacggcagac gttcccaaat atacgatcgg aagcacgcgt 420
   cacgaaccaa aacctgctac accggctacc gcctctccta cgatccagca ttcacaccca 480
   ggagcacctc accaccatca ccaccatcac catcaccacc ctcatggtac aacggcaccg 540
   acaccagcct caactcctgc taccgccgca acttccacga caccggcgac tgccagcgca 600
   tcacttccca agcgcgaagc cccattcaat tacggctatc gaagtatgtt tggtgggtcg 660
   ggattatcag agcaagatct gagggaacgt gaaagggctc gtcagcgtga gcgagagagg 720
   gaacaggcga ggattgagca ggagagaaag gatagagcgg ccaaggcgaa cccagcgaca 780
   tcaccacgcg catccactgg gacaccggca aacccatacc ctcgtccatc acttccaccg 840
   tcgcctacga atgcccgctc tttaccatcc gcggcatccc cagtgattcc caacaaaccc 900
   tcccccgctc tacctccaac agcaaccgct accggctcaa gccagcgccc gaatctaccc 960
   ctaccaggac tcgctgccat cggcggccga tccttaccct caccattcga tccccaacga 1020
   gaacgtccac catcagcaac ggctccacaa gctcctggtg tagctcaccg tcgcacgccc 1080
   agtggaggga ctggaacacg ccccgatcct gctgctttgg gccccggcca gctcagtcca 1140
   ggcaaatccg caacatctca acctggcgct cgatccgctt tcggtacgtc cgcctttgga 1200
   ggagctcgtg atgccaagtc gacgcccaac acctcccaac ctcccctccc atcggctcgt   1260
   gaaccccctc gatccgcttc tgccgcctct gccgctccca aacccgcaac agctgccacc   1320
   tcgtcagcag cacgaccacc tggacgagat ttccccggtt ccaactatcc cggatacggc 1380
   gggttcatgc cattcagtgg aggattcgag tatggatcgt ttggtggatg gaccggtaac 1440
   tcggctagcc atgctgctca aagagcacat acgacggcag agcaagatcg attcgatcga 1500
   gagaggaaac gacttcagga tgaacaagaa gctcgtatgg ctcgtgtgcg tgctgcggct 1560
   gctcagaggg aaaaggaagt ccgagaagct agggaagcta aagagaggca aaaggagcag 1620
   gaacgactgg catatgctcg acaagccgag atcaattcag cacgtatcaa agaagcaaac 1680
   caacgtatgg atcaagcgaa tcgggatcaa ttcaggcgat acctcgacgg taatcctgcc 1740
   catcctgcag cgcagactcc gcatgatgct ctgcagcaag tcgcccctag gcgagaacca 1800
   cgtccgtatg aatacaagac ggattcaagg gattatcaat acactccaag ggataaacga 1860
   cctcgaatgg atgctgccgt cgacgatgct aggcgaggaa atgccaacaa gtcgaaaagg 1920
   cgtaaagacg aggaccgacc taaatcaccc gttcgaatac gcgacttcag tcatttgacc 1980
   gaggccacac gtaaatatcc cgatgtcaag agtaccgcga tcgaggcatg gctcaagacg 2040
   aaatcggaaa tgaatcgggt tgtagctacc gagacgtata ccggttcgga ttatcgttta 2100
   tctcgtcacc gttctcgaaa tctacagggt ggactcgtca aggtccacat aggtgggggg 2160
   ttccttggga agaactggaa attgagagga gcacctggat gggaagatgc atcctcgtgt 2220
   ccggtcaaga tccgtgaagg tgttggcaaa gggcgtgggg agattagtgc cggatgggag 2280
   aagaggggga tctggggtac ggatgtttat accgatgact ctgacctggg gcttgtcctg 2340
   gtccatgccg gctggatcat gtggggtgga cagggaccgt ccacggataa cgatggcgat 2400
   acactggtgg tggatgtccg gatcgttccc ccactcgtcc attataccgc taccgaccga 2460
   tgcggcgttc tcactaggaa ttgggggaac ggacatgatg gatccagtat cgtcattgaa 2520
   gccgtcaagc gtgttcccgt gagtcccctc tttttcctct caaaccaaac t 2571
<210> 9
   <211> 912
   <212> DNA
   <213> Virus
<400> 9
   ctcaccgtcg cacgcccagt ggagggactg gaacacgccc cgatcctgct gctttgggcc      60
   ccggccagct cagtccaggc aaatccgcaa catctcaacc tggcgctcga tccgctttcg     120
   gtacgtccgc ctttggagga gctcgtgatg ccaagtcgac gcccaacacc tcccaacctc     180
   ccctcccatc ggctcgtgaa ccccctcgat ccgcttctgc cgcctctgcc gctcccaaac     240
   ccgcaacagc tgccacctcg tcagcagcac gaccacctgg acgagatttc cccggttcca     300
   actatcccgg atacggcggg ttcatgccat tcagtggagg attcgagtat ggatcgtttg     360
   gtggatggac cggtaactcg gctagccatg ctgctcaaag agcacatacg acggcagagc     420
   aagatcgatt cgatcgagag aggaaacgac ttcaggatga acaagaagct cgtatggctc     480
   gtgtgcgtgc tgcggctgct cagagggaaa aggaagtccg agaagctagg gaagctaaag     540
   agaggcaaaa ggagcaggaa cgactggcat atgctcgaca agccgagatc aattcagcac     600
   gtatcaaaga agcaaaccaa cgtatggatc aagcgaatcg ggatcaattc aggcgatacc     660
   tcgacggtaa tcctgcccat cctgcagcgc agactccgca tgatgctctg cagcaagtcg     720
   cccctaggcg agaaccacgt ccgtatgaat acaagacgga ttcaagggat tatcaataca     780
   ctccaaggga taaacgacct cgaatggatg ctgccgtcga cgatgctagg cgaggaaatg     840
   ccaacaagtc gaaaaggcgt aaagacgagg accgacctaa atcacccgtt cgaatacgcg     900
   acttcagtca tt                                                         912
<210> 10
   <211> 877
   <212> PRT
   <213> Virus
<400> 10
   Pro Asn Ser Gln Gln His Thr Leu Lys Glu Pro Ser Pro Arg Arg Leu
   1               5                   10                  15
   Ala Ile Arg Leu Pro Thr Pro Pro Ala Ser Gln Ser Gly Thr Asp Val
               20                  25                  30
   Glu Glu Thr Ile Ser Arg Thr Leu Val Arg Pro Arg Pro Ala Pro Thr
           35                  40                  45
   Pro Pro Arg Arg Ser Ala Gly Pro Ser Ile Leu Leu Asn Gly Met Ala
       50                  55                  60
   Ser Pro Thr Gln Asp Ile Ser Pro Asn Pro Arg Arg Lys Lys Ser Pro
   65                  70                  75                  80
   Arg Val Asp Glu Ala Gly Pro Ser Gln Leu Arg Ser Pro Phe Glu Ile
                   85                  90                  95
   Ala Pro Thr Thr Thr Ser Pro Arg His Pro Arg Asp Arg Val Glu Ile
               100                 105                 110
   Val Ile Gly Gln Pro Ser Arg Lys Ser Val Ser Pro Ser Ala Thr Pro
           115                 120                 125
   Lys Ala Ser Lys Pro Arg Lys Ser Arg Val Ser Thr Lys Thr Pro Ala
       130                 135                 140
   Ser His Pro Leu Ser Gln Ser Thr Ser Ser Gln Pro Ser Arg Ala Lys
   145                 150                 155                 160
   Arg Ala Pro Ala Thr Ala Pro Gln Pro Asn Arg Arg Lys Ser Thr Pro
                   165                 170                 175
   Ala Thr Arg Lys Pro Ser Pro Pro Arg Thr Thr Ala Lys Pro Lys Phe
               180                 185                 190
   Ala Lys Thr Pro Lys Arg Leu Ser Leu Pro Gly Arg Gly Arg Ala Arg
           195                 200                 205
   Lys Ser Val Thr Pro Ala Glu Leu Lys Ala Met Ile Gly Leu Leu Pro
       210                 215                 220
   Glu Pro Val Glu Gln Ser Ser Pro Ser Asp Asp Pro Leu Leu Leu Val
   225                 230                 235                 240
   Ser Asp Asp Glu Asp His Thr Pro Ala Ile Arg Pro Ser Ser His Ser
   245 250 255
   Gly Lys Ser Thr Gly Ser Arg Ser Ala Ala Arg Ile Ile Asp Phe Asp
   260 265 270
   Ala Ala Ile Gln Gly Arg Ser Ser Val Glu Glu Val Val Arg Asp Tyr
   275 280 285
   Glu Val Pro Ile His Gly Ser Glu Pro Asp Tyr Asp Val Ala Arg Asp
   290 295 300
   Tyr Gly Phe Asp Ser Gly Asp Ser Asp Glu Glu Met Gly Asn Asp Thr
   305 310 315 320
   Phe Val Asn Val Ala Gly Ala Arg Ala Lys Val Asn Pro Ala Asp His
   325 330 335
   Gln Asp Glu Ala Thr Leu Glu Asp Glu Asp Glu Asn Gly Ser His Leu
   340 345 350
   Ser Ser Arg Ser Asn Ser Pro Phe Ala Phe Gln Glu Ala Glu Val Arg
   355 360 365
   Phe Ser Ser Pro Ala Leu Ser Leu Arg Ala Ala Ser Pro Ala Lys Glu
   370 375 380
   Asp Ser Arg Leu Val Ser Ser Pro Val Ile Ala Ser Val Ala Ser Pro
   385 390 395 400
   Val Pro Ser Val Ala Ala Ser Leu Pro Ser Pro Phe Leu Ser Arg Ser
   405 410 415
   Thr Asn Ser Pro Arg Leu Ser Arg Ser Thr Thr Ala Thr Pro Val Val
   420 425 430
   His Ser His Gly Leu Pro Ser Pro Ala Pro Ser Ser Pro Pro Pro Thr
   435 440 445
   Glu Met Glu Pro Glu Val Ala Ser Ser Pro Arg Tyr Ala Arg Ser Gln
   450 455 460
   Thr Gly Ile Pro Arg Ala Thr Val Ala Val Asn Gln Glu Leu Glu Ser
   465 470 475 480
   Ala Thr Val Pro Ala Ser Pro Ala Tyr Ser Glu Lys Phe Ser Val Gly
   485 490 495
   Met Leu Ser Ala Pro Ser Ser Pro Phe Ser Thr Arg Ser Arg Asp Lys
   500 505 510
   Ser Ala Thr Pro Arg Phe Thr Glu Gln Leu Ser Val His Ser Gln Gly
   515 520 525
   Met Asp Glu Glu Glu Val Gln Asp Pro Cys Ser Ala Ser Pro Leu Leu
   530 535 540
   Leu Pro Gly Thr Pro Ser Ala Leu Gly Leu Phe Met Pro Glu Glu Ala
   545 550 555 560
   Asp Gly Met Ser Thr Pro Tyr Ser His Ile Gly Ala Glu Pro Asp Thr
   565 570 575
   Pro Ala Pro Asn Phe Asp Ser Pro Ser Arg Ser Ser Ile His Met Asp
   580 585 590
   Ser Pro Ala Pro Val Glu Ala Arg Thr Ser Pro Thr Leu Phe Gly Ser
   595 600 605
   Arg Val Ala Ser Pro Ala Arg Ser Val Val Ala Gln Val Ala Ser Pro
   610 615 620
   Val Arg Pro Met Ala Thr Gln Val Ala Ser Pro Val Arg Ser Ser Ala
   625 630 635 640
   Ser Gln Met Asp Ser Pro Ala Arg Ser Val Ser Arg Ala Gly Ser Pro
   645 650 655
   Ala Leu Ser Ser Ala Ser Arg Ile Gly Ser Pro Ala Arg Pro Val Thr
   660 665 670
   Leu Gln Ala Ala Ser Pro Ala Arg Ser Thr Thr Ser Gln Ala Ala Glu
   675 680 685
   Leu Ser Met Gly Tyr Ser Gln Ser Pro Arg Arg Ser Pro Val Cys Leu
   690 695 700
   Pro Gln Glu Met Asp Leu Pro Glu Glu Ile Pro Thr Val Ser Thr Ser
   705 710 715 720
   Ser Pro Arg Arg Ser Pro Val Cys Leu Pro Ser His Val Asp Val Val
   725 730 735
   Asp Asn Val Glu Glu Gln Pro Leu Pro Val Val Asn Thr His Ser Phe
   740 745 750
   Arg Lys Ser Pro Leu Ala Tyr Ala Gln Ser Pro Arg Arg Ser Pro Val
   755 760 765
   Cys Leu Pro Ala Asp Met Glu Asn Ala Asp Glu Asp Glu Ala Gly Asp
   770 775 780
   Lys Glu Asn Met Ala Pro Val Gln Ser Thr Thr Pro Ile Leu Ser Pro
   785 790 795 800
   Pro Thr Leu Lys Ser Thr Pro Pro Met Tyr Ala Phe Thr Phe Ser Pro
   805 810 815
   Arg Ile Ala Ser Pro Leu Arg Ile Ala His Asn Gln Ala Ser Ala Leu
   820 825 830
   Lys Arg Asp Ser Ile Ala Arg Thr Ser Gln Ser Pro Ile Pro Ala Val
   835 840 845
   Glu Ser Phe His Glu Arg Phe Ala Glu Val Asp Glu Cys Pro Ser Ala
   850 855 860
   Asp Val Thr Leu Glu Gly Asn Ser Ala Asp Trp Ser Leu
   865 870 875
<210> 11
   <211> 145
   <212> PRT
   <213> Virus
<400> 11
   Glu Glu Lys Glu Gly Thr His Gly Asn Thr Leu Asp Gly Phe Asn Asp
   1 5 10 15
   Asp Thr Gly Ser Ile Met Ser Val Pro Pro Ile Pro Ser Glu Asn Ala
   20 25 30
   Ala Ser Val Gly Ser Gly Ile Met Asp Glu Trp Gly Asn Asp Pro Asp
   35 40 45
   Ile His His Gln Cys Ile Ala Ile Val Ile Arg Gly Arg Ser Leu Ser
   50 55 60
   Thr Pro His Asp Pro Ala Gly Met Asp Gln Asp Lys Pro Gln Val Arg
   65 70 75 80
   Val Ile Gly Ile Asn Ile Arg Thr Pro Asp Pro Pro Leu Leu Pro Ser
   85 90 95
   Gly Thr Asn Leu Pro Thr Pro Phe Ala Asn Thr Phe Thr Asp Leu Asp
   100 105 110
   Arg Thr Arg Gly Cys Ile Phe Pro Ser Arg Cys Ser Ser Gln Phe Pro
   115 120 125
   Val Leu Pro Lys Glu Pro Pro Thr Tyr Val Asp Leu Asp Glu Ser Thr
   130 135 140
   Leu
   145
<210> 12
   <211> 287
   <212> PRT
   <213> Virus
<400> 12
   Pro Asn Val Ala Leu Leu Ile Gly Val Thr Ala Phe Phe His Ser Thr
   1               5                   10                  15
   Ser Thr Val Asn Lys Gln Leu Ser Tyr Thr Leu Ile Leu Ile His Ser
               20                  25                  30
   Phe Asp His Thr Tyr Cys Tyr Thr His Thr Arg Ser Ala Cys Leu Thr
           35                  40                  45
   Trp Leu Leu Ser Gln Gln Leu Asp His Asp Ala Tyr Gln Pro Ser Arg
       50                  55                  60
   Asn Thr Arg Gln His Arg Asn His Lys Val Asn Asn Arg Ser Glu Thr
   65                  70                  75                  80
   Ser His Ser Thr Asp Ser Tyr Thr Met Ser Arg Glu Leu Val Ser Ser
                   85                  90                  95
   Ser Ser Tyr Pro Leu Ser Asn Pro Thr Pro Ser Ser Ser Met Lys Ser
               100                 105                 110
   Ser Gln Ser Arg Ile Pro Val Lys Ala Lys Arg Ala Pro Leu Gly Glu
           115                 120                 125
   Arg Val Asp Asn His Asp His Thr Thr Pro Arg Gln His Leu Val Lys
       130                 135                 140
   Ser Val Lys Ser Val Ile Arg Pro Arg Ile Ile Ser Thr Lys Ser Thr
   145                 150                 155                 160
   Ala Ser Pro Ser Lys Ser Ser Thr Tyr Arg Pro Ser Pro Arg Ala Ala
                   165                 170                 175
   Val Gln Gly Ser Pro Ser Ser Ser Ile Val Ser Ser Pro Ala Gly Ala
               180                 185                 190
   Asn Val Ser Arg Pro Gly His Thr Pro His Pro Arg Arg Ser Ser Val
           195                 200                 205
   Gly Leu Ala Phe Leu Gly His Asp Val Ser Met Leu Met Asp Thr Thr
       210                 215                 220
   Arg Leu Asp Phe Val Ser Asp Thr Asp Asp Ser Glu Gly Asp Glu Pro
   225                 230                 235                 240
   Leu Asp Asn Met Leu Val Arg Arg Gly Val Met Gly Pro Gly Arg Leu
                   245                 250                 255
   Met Thr Pro Ala Asn Ser Gln Glu Ser Gln Val Ser Ser Leu Cys Leu
               260                 265                 270
   Leu Glu Ser Gln Leu Thr Cys Ser Pro Thr Leu Ser Ser Ile Leu
           275                 280                 285
<210> 13
   <211> 328
   <212> PRT
   <213> Virus
<400> 13
   Thr Arg Ser Ser Ser Pro Leu Pro Tyr Gln Leu Leu Pro Pro Thr Ala
   1               5                   10                  15
   Lys Ser Ser Leu Leu Val Cys Ser Gln Leu Pro Arg Leu Arg Ser Leu
               20                  25                  30
   Pro Asp His Ala Ile Asn Pro Pro Arg Leu Asp Ser Gln Asn Asn Tyr
           35                  40                  45
   Gln Tyr Ile Arg Arg Glu Trp Met Lys Arg Lys Tyr Arg Ile His Val
       50                  55                  60
   Val Leu Val His Tyr Ser Tyr Leu Glu His His Pro Pro Leu Val Ser
   65                  70                  75                  80
   Leu Cys Leu Lys Lys Arg Met Gly Cys Arg Leu His Thr Pro Thr Leu
                   85                  90                  95
   Gly Arg Ser Leu Ile Leu Leu Pro Gln Thr Leu Ile Arg Pro His Gly
               100                 105                 110
   Gln Ala Tyr Ile Trp Thr Arg Gln Leu Gln Ser Lys Leu Gly His Arg
           115                 120                 125
   Gln Leu Ser Leu Val His Gly Ser His Arg Gln Pro Ala Pro Leu Leu
       130                 135                 140
   Leu Lys Ser Leu Arg Pro Ser Asp Leu Trp Leu Leu Arg Ser His His
   145                 150                 155                 160
   Leu Phe Val Leu Leu Leu Leu Arg Trp Ile Arg Leu Leu Ala Leu Phe
                   165                 170                 175
   Leu Glu Leu Asp Arg Leu Pro Phe Leu Leu Leu Leu Glu Leu Asp Arg
               180                 185                 190
   Leu Pro Asp Gln Ser Leu Tyr Arg Leu His Arg Leu Leu Val Pro Leu
           195                 200                 205
   Leu Pro Arg Gln Leu Ser Cys Pro Trp Ala Ile Leu Asn His Arg Glu
       210                 215                 220
   Asp Leu Arg Ser Val Tyr Leu Lys Arg Trp Ile Tyr Pro Lys Lys Tyr
   225                 230                 235                 240
   Arg Pro Ser Ala Pro Ala Arg Pro Gly Gly Pro Arg Ser Ala Ser Leu
                   245                 250                 255
   Leu Met Trp Met Leu Trp Thr Thr Ser Arg Ser Ser His Tyr Gln Ser
               260                 265                 270
   Ser Thr Pro Ile Arg Leu Gly Ser Arg His Ser Pro Thr Pro Asn His
           275                 280                 285
   Leu Glu Gly Leu Gln Ser Ala Tyr Pro Pro Thr Trp Arg Met Pro Thr
       290                 295                 300
   Lys Met Lys Leu Gly Ile Lys Arg Thr Trp Arg Leu Phe Asn Arg Leu
   305                 310                 315                 320
   Leu Gln Ser Tyr His Pro Leu His
                   325
<210> 14
   <211> 540
   <212> PRT
   <213> Virus
<400> 14
   Arg Gly Thr Val Arg Ile Gly Val Cys Ser Asp Glu Gly Leu Pro Glu
   1 5 10 15
   Val Asp Arg Glu Glu His Gly Leu Gly Leu Asp Leu Gln Gly Glu Asp
   20 25 30
   Gln Glu Asn Asp Asp Asn Leu Ser Ile Ser Leu Ser Ile Ala Glu Glu
   35 40 45
   Gly Glu Glu Glu Glu Glu Ser Asp Leu Val Asp Ser Ser Ile Glu Gln
   50 55 60
   Glu Asp Asp Leu Glu Leu Gly Ala Asp Pro Thr Thr Gln Leu Ala Asp
   65 70 75 80
   Asp Ile Val Asp Ser Asn Glu Asp Glu Gln Asp Ala Glu Glu Asp Ala
   85 90 95
   Glu Gly Ser Glu Glu Glu Glu Gly Gln Glu Ser Asn Val Ile Ala Pro
   100 105 110
   Ile Pro Thr Phe Thr Asp Glu Arg Asn Ala Ala Glu Glu Gly Glu Ser
   115 120 125
   Thr Asp Ser Glu Glu Ala Pro Ser Ser Asp Asp Glu Gln Glu Asp Glu
   130 135 140
   Thr Ala Gln Glu Glu Leu Glu Glu Asp Ile Pro Ala Ser Pro Ala Pro
   145 150 155 160
   Val Lys Ile Val Leu Lys Leu Ile Asn Arg Gly Ile Val Lys Ile Glu
   165 170 175
   Pro Pro Ala Ser Pro Ala Arg Ala Asn Glu Ala Asp Ala Ser Glu Leu
   180 185 190
   Arg Ser Thr Thr Pro Ala Tyr Asp Pro Pro Gln Pro Ile Val Ala Ala
   195 200 205
   Pro Met Thr Arg Ser Pro Ala Pro Glu Ala Thr Thr Val Tyr Pro Ala
   210 215 220
   Leu Pro Thr Thr Pro Ile Thr Ala Pro Leu Ser Pro Leu Val Ser Thr
   225 230 235 240
   Thr Pro Leu Gly Thr Pro Pro Ala Lys Gln Arg Ser Pro Ala Leu Gln
   245 250 255
   Gln Ser Leu Pro Gln Ser Ser Asp Tyr Ser Thr Leu Gln Ala Gly Pro
   260 265 270
   Ser Thr Arg Pro Arg Ser Arg Leu Ser Arg Gln Ile Pro Ala Ser Pro
   275 280 285
   Glu Gln Glu Glu Ser Asp Ser Ile Arg Val Arg Thr Pro Arg Ser Leu
   290 295 300
   Gly Leu Glu Leu Glu Asp Ala Glu Ala Asp Asn Ser Met Arg Ser Val
   305 310 315 320
   Val Glu Val Ser Ser Leu Asp Pro Lys Ala Ala Ala Arg Ala Ala Ala
   325 330 335
   Ile Leu Lys Leu Val Ser Phe Phe Asp Ser Leu His Gly Gln Ala Asp
   340 345 350
   Met Gln Asn His Ala Tyr Ile Glu His Gly Asp Ile Ser Lys Leu Gly
   355 360 365
   Glu Ser Thr Ala Ala Arg His Gln Ser Met His Ser Ala Arg Leu Asp
   370 375 380
   Lys Leu Asp Ala Ser Arg Arg Glu Asp Lys Thr Glu Leu Leu Tyr Glu
   385 390 395 400
   Ala Glu Leu Glu Ile Val Ser Thr Arg Arg Ser Arg Ser Arg Ser Met
   405 410 415
   Ser Arg Phe Arg Gly Ile Ser Glu Ala Pro Thr Glu Leu Pro Leu Pro
   420 425 430
   Gly Ala Trp Asn Gly Thr Pro Lys Arg Lys Arg Ser Ala His Leu Gln
           435                 440                 445
   Ile Glu Ala Pro Lys Thr Ser Asn Thr Trp Gly Val Ser Gln Trp Lys
       450                 455                 460
   Lys Leu Glu Lys Val Phe Arg Ala Glu Arg Glu Ile Trp Val Thr Glu
   465                 470                 475                 480
   Arg Asn Val Lys Ala Met Pro Gly Gly Phe Ile Gly Trp Ala Arg Met
                   485                 490                 495
   Ser Thr Phe Gly Pro Pro Ala Ala Val Ala Val Pro Trp Asp Pro Thr
               500                 505                 510
   Arg Val Val Asp Arg Phe Leu Glu Glu Gln Gly Ile Ser Lys Lys Glu
           515                 520                 525
   Gln Val Gly Asp Trp Ser Arg Tyr Val His Leu Pro
       530                 535                 540
<210> 15
   <211> 243
   <212> PRT
   <213> Virus
<400> 15
   Arg Glu Leu Met Asn Arg Glu Met Ile Tyr Thr Arg Val Glu Ala Leu
   1               5                   10                  15
   Glu Arg His Ala Glu His Arg Lys Ile Lys Glu Thr Ser Gln Glu Glu
               20                  25                  30
   Arg Ser Pro Lys Lys Pro Lys Thr Ala Pro Ala Pro Pro Ser Thr Val
           35                  40                  45
   Arg Lys Leu Phe Gly Trp Val Met Pro Ser Ala Thr Lys Ser Ala Leu
       50                  55                  60
   Pro Val Ala Ala Pro Glu Ser Ser Val Lys Asn Met Asp Lys Gly Lys
   65                  70                  75                  80
   Gly Lys Glu Arg Glu Gly Arg Gln Thr Phe Lys Asp Arg Leu Glu Ala
                   85                  90                  95
   Leu Gln Asp Gln Pro Met Thr Gln Leu Ser Glu Ser Val Ser Ile Pro
               100                 105                 110
   Arg Pro Thr Pro Ile Ala Ser Ser Thr Pro Asn Thr Ala Pro Pro His
           115                 120                 125
   Pro Ser Ser Pro Ala Trp Thr Asn Ala Tyr Gln Pro Ile Ser Thr Ala
       130                 135                 140
   Asn Ala His Ala Ser Gly Ser Thr Thr Thr Arg Pro Thr Pro Arg Thr
   145                 150                 155                 160
   Leu Ser Ala Ile Leu Ala Glu Ser Ser Ser Thr Thr Ser Ser Arg Gly
                   165                 170                 175
   Gln Leu His Ser Ser Leu Ser Gln Arg Ser Ala Ala Leu Asp Ala Leu
               180                 185                 190
   Phe Gly Gly Ser Thr Ser Thr Ser Thr Arg Pro Val Val Gln Ile Lys
           195                 200                 205
   Arg Ser Ser Ser Val Lys Asp Ile Val Arg Gly Phe Glu Asp Ser Gly
       210                 215                 220
   Ala Leu Ala Lys Ser Thr Gly Glu Glu Gly Leu Arg Arg Val Gln Ser
   225                 230                 235                 240
   Arg Pro Leu
<210> 16
   <211> 857
   <212> PRT
   <213> Virus
<400> 16
   Thr Tyr Asp Pro Pro Glu Arg Pro Thr Thr Met Pro Ser Pro His Ala
   1               5                   10                  15
   Ser Thr Asn Pro Gln Pro Lys Pro Thr Asp Pro Leu Pro Gln Arg Pro
               20                  25                  30
   Gly Thr Asn Leu Pro Gln Thr Ser Gly Pro Pro Arg Ala Glu Glu Ala
                   35                  40                  45
   Thr Ile Ile Arg Ser Phe Ser Asn Phe Gly Gly Leu Gln Arg Pro Arg
       50                  55                  60
   Asn Pro Ala Ser Ser Ser Pro Ser Leu Thr Gly Ala Gly Leu Gly Asp
   65                  70                  75                  80
   Thr Gly Ala Arg Pro Gly Asn Asn Ser Ala Ser Ser Leu Gly Gly Pro
                   85                  90                  95
   Gly Asn Gly Leu Arg Pro Ser Tyr Ser Pro Phe Gly Ser Arg Asp Pro
               100                 105                 110
   Pro Pro Val Ala Gly Glu Leu Thr Ile Gly Glu Met Ser Glu Leu Thr
           115                 120                 125
   Ala Asp Val Pro Lys Tyr Thr Ile Gly Ser Thr Arg His Glu Pro Lys
       130                 135                 140
   Pro Ala Thr Pro Ala Thr Ala Ser Pro Thr Ile Gln His Ser His Pro
   145                 150                 155                 160
   Gly Ala Pro His His His His His His His His His His Pro His Gly
                   165                 170                 175
   Thr Thr Ala Pro Thr Pro Ala Ser Thr Pro Ala Thr Ala Ala Thr Ser
               180                 185                 190
   Thr Thr Pro Ala Thr Ala Ser Ala Ser Leu Pro Lys Arg Glu Ala Pro
           195                 200                 205
   Phe Asn Tyr Gly Tyr Arg Ser Met Phe Gly Gly Ser Gly Leu Ser Glu
       210                 215                 220
   Gln Asp Leu Arg Glu Arg Glu Arg Ala Arg Gln Arg Glu Arg Glu Arg
   225                 230                 235                 240
   Glu Gln Ala Arg Ile Glu Gln Glu Arg Lys Asp Arg Ala Ala Lys Ala
                   245                 250                 255
   Asn Pro Ala Thr Ser Pro Arg Ala Ser Thr Gly Thr Pro Ala Asn Pro
               260                 265                 270
   Tyr Pro Arg Pro Ser Leu Pro Pro Ser Pro Thr Asn Ala Arg Ser Leu
           275                 280                 285
   Pro Ser Ala Ala Ser Pro Val Ile Pro Asn Lys Pro Ser Pro Ala Leu
       290                 295                 300
   Pro Pro Thr Ala Thr Ala Thr Gly Ser Ser Gln Arg Pro Asn Leu Pro
   305                 310                 315                 320
   Leu Pro Gly Leu Ala Ala Ile Gly Gly Arg Ser Leu Pro Ser Pro Phe
                   325                 330                 335
   Asp Pro Gln Arg Glu Arg Pro Pro Ser Ala Thr Ala Pro Gln Ala Pro
               340                 345                 350
   Gly Val Ala His Arg Arg Thr Pro Ser Gly Gly Thr Gly Thr Arg Pro
           355                 360                 365
   Asp Pro Ala Ala Leu Gly Pro Gly Gln Leu Ser Pro Gly Lys Ser Ala
       370                 375                 380
   Thr Ser Gln Pro Gly Ala Arg Ser Ala Phe Gly Thr Ser Ala Phe Gly
   385                 390                 395                 400
   Gly Ala Arg Asp Ala Lys Ser Thr Pro Asn Thr Ser Gln Pro Pro Leu
                   405                 410                 415
   Pro Ser Ala Arg Glu Pro Pro Arg Ser Ala Ser Ala Ala Ser Ala Ala
               420                 425                 430
   Pro Lys Pro Ala Thr Ala Ala Thr Ser Ser Ala Ala Arg Pro Pro Gly
           435                 440                 445
   Arg Asp Phe Pro Gly Ser Asn Tyr Pro Gly Tyr Gly Gly Phe Met Pro
       450                 455                 460
   Phe Ser Gly Gly Phe Glu Tyr Gly Ser Phe Gly Gly Trp Thr Gly Asn
   465                 470                 475                 480
   Ser Ala Ser His Ala Ala Gln Arg Ala His Thr Thr Ala Glu Gln Asp
                   485                 490                 495
   Arg Phe Asp Arg Glu Arg Lys Arg Leu Gln Asp Glu Gln Glu Ala Arg
               500                 505                 510
   Met Ala Arg Val Arg Ala Ala Ala Ala Gln Arg Glu Lys Glu Val Arg
           515                 520                 525
   Glu Ala Arg Glu Ala Lys Glu Arg Gln Lys Glu Gln Glu Arg Leu Ala
       530                 535                 540
   Tyr Ala Arg Gln Ala Glu Ile Asn Ser Ala Arg Ile Lys Glu Ala Asn
   545                 550                 555                 560
   Gln Arg Met Asp Gln Ala Asn Arg Asp Gln Phe Arg Arg Tyr Leu Asp
   565 570 575
   Gly Asn Pro Ala His Pro Ala Ala Gln Thr Pro His Asp Ala Leu Gln
   580 585 590
   Gln Val Ala Pro Arg Arg Glu Pro Arg Pro Tyr Glu Tyr Lys Thr Asp
   595 600 605
   Ser Arg Asp Tyr Gln Tyr Thr Pro Arg Asp Lys Arg Pro Arg Met Asp
   610 615 620
   Ala Ala Val Asp Asp Ala Arg Arg Gly Asn Ala Asn Lys Ser Lys Arg
   625 630 635 640
   Arg Lys Asp Glu Asp Arg Pro Lys Ser Pro Val Arg Ile Arg Asp Phe
   645 650 655
   Ser His Leu Thr Glu Ala Thr Arg Lys Tyr Pro Asp Val Lys Ser Thr
   660 665 670
   Ala Ile Glu Ala Trp Leu Lys Thr Lys Ser Glu Met Asn Arg Val Val
   675 680 685
   Ala Thr Glu Thr Tyr Thr Gly Ser Asp Tyr Arg Leu Ser Arg His Arg
   690 695 700
   Ser Arg Asn Leu Gln Gly Gly Leu Val Lys Val His Ile Gly Gly Gly
   705 710 715 720
   Phe Leu Gly Lys Asn Trp Lys Leu Arg Gly Ala Pro Gly Trp Glu Asp
   725 730 735
   Ala Ser Ser Cys Pro Val Lys Ile Arg Glu Gly Val Gly Lys Gly Arg
   740 745 750
   Gly Glu Ile Ser Ala Gly Trp Glu Lys Arg Gly Ile Trp Gly Thr Asp
   755 760 765
   Val Tyr Thr Asp Asp Ser Asp Leu Gly Leu Val Leu Val His Ala Gly
   770 775 780
   Trp Ile Met Trp Gly Gly Gln Gly Pro Ser Thr Asp Asn Asp Gly Asp
   785 790 795 800
   Thr Leu Val Val Asp Val Arg Ile Val Pro Pro Leu Val His Tyr Thr
   805 810 815
   Ala Thr Asp Arg Cys Gly Val Leu Thr Arg Asn Trp Gly Asn Gly His
   820 825 830
   Asp Gly Ser Ser Ile Val Ile Glu Ala Val Lys Arg Val Pro Val Ser
   835 840 845
   Pro Leu Phe Phe Leu Ser Asn Gln Thr
   850 855
<210> 17
   <211> 304
   <212> PRT
   <213> Virus
<400> 17
   Leu Thr Val Ala Arg Pro Val Glu Gly Leu Glu His Ala Pro Ile Leu
   1 5 10 15
   Leu Leu Trp Ala Pro Ala Ser Ser Val Gln Ala Asn Pro Gln His Leu
   20 25 30
   Asn Leu Ala Leu Asp Pro Leu Ser Val Arg Pro Pro Leu Glu Glu Leu
   35 40 45
   Val Met Pro Ser Arg Arg Pro Thr Pro Pro Asn Leu Pro Ser His Arg
   50 55 60
   Leu Val Asn Pro Leu Asp Pro Leu Leu Pro Pro Leu Pro Leu Pro Asn
   65 70 75 80
   Pro Gln Gln Leu Pro Pro Arg Gln Gln His Asp His Leu Asp Glu Ile
   85 90 95
   Ser Pro Val Pro Thr Ile Pro Asp Thr Ala Gly Ser Cys His Ser Val
   100 105 110
   Glu Asp Ser Ser Met Asp Arg Leu Val Asp Gly Pro Val Thr Arg Leu
   115 120 125
   Ala Met Leu Leu Lys Glu His Ile Arg Arg Gln Ser Lys Ile Asp Ser
   130 135 140
   Ile Glu Arg Gly Asn Asp Phe Arg Met Asn Lys Lys Leu Val Trp Leu
   145 150 155 160
   Val Cys Val Leu Arg Leu Leu Arg Gly Lys Arg Lys Ser Glu Lys Leu
   165 170 175
   Gly Lys Leu Lys Arg Gly Lys Arg Ser Arg Asn Asp Trp His Met Leu
   180 185 190
   Asp Lys Pro Arg Ser Ile Gln His Val Ser Lys Lys Gln Thr Asn Val
   195 200 205
   Trp Ile Lys Arg Ile Gly Ile Asn Ser Gly Asp Thr Ser Thr Val Ile
   210 215 220
   Leu Pro Ile Leu Gln Arg Arg Leu Arg Met Met Leu Cys Ser Lys Ser
   225 230 235 240
   Pro Leu Gly Glu Asn His Val Arg Met Asn Thr Arg Arg Ile Gln Gly
   245 250 255
   Ile Ile Asn Thr Leu Gln Gly Ile Asn Asp Leu Glu Trp Met Leu Pro
   260 265 270
   Ser Thr Met Leu Gly Glu Glu Met Pro Thr Ser Arg Lys Gly Val Lys
   275 280 285
   Thr Arg Thr Asp Leu Asn His Pro Phe Glu Tyr Ala Thr Ser Val Ile
   290 295 300
<210> 18
   <211> 20
   <212> DNA
   <213> Artificial Sequence
<220>
   <223> primer 101-C
<400> 18
   caacaccgca atcacaaagt 20
<210> 19
   <211> 20
   <212> DNA
   <213> Artificial Sequence
<220>
   <223> primer N101-B
<400> 19
   aacattgaaa cgtcatgtcc 20
<210> 20
   <211> 20
   <212> DNA
   <213> Artificial Sequence
<220>
   <223> primer KS-2
<400> 20
   ctcgtctdgt cgtcatcgta 20
<210> 21
   <211> 21
   <212> DNA
   <213> Artificial Sequence
<220>
   <223> primer N101-D
<400> 21
   catttgctcc cgctggagat g 21
<210> 22
   <211> 23
   <212> DNA
   <213> Artificial Sequence
<220>
   <223> primer K-5
<400> 22
   aacgatccgg acatccacca cca 23
<210> 23
   <211> 20
   <212> DNA
   <213> Artificial Sequence
<220>
   <223> primer KIV-21
<400> 23
   gcgatcgagg catggctcaa 20
<210> 24
   <211> 20
   <212> DNA
   <213> Artificial Sequence
<220>
   <223> primer 101-1a
<400> 24
   ctgcagagca tcatgcggag 20
<210> 25
   <211> 20
   <212> DNA
   <213> Artificial Sequence
<220>
   <223> primer 101-T
<400> 25
   tcgatccgct ttcggtacgt 20
<210> 26
   <211> 20
   <212> DNA
   <213> Artificial Sequence
<220>
   <223> primer X-2
<400> 26
   acctaatgaa gacgccgaat 20
<210> 27
   <211> 20
   <212> DNA
   <213> Artificial Sequence
<220>
   <223> primer KS-1
<400> 27
   gagacatggt gtaagagtcg 20
<210> 28
   <211> 20
   <212> DNA
   <213> Artificial Sequence
<220>
   <223> primer 101-11
<400> 28
   agtggatgaa gctggacctt 20
<210> 29
   <211> 20
   <212> DNA
   <213> Artificial Sequence
<220>
   <223> primer Op-3
<400> 29
   gatcttgatc ctgtactctt 20
<210> 30
   <211> 20
   <212> DNA
   <213> Artificial Sequence
<220>
   <223> primer 101-21
<400> 30
   gcgaaagagg attctcgact 20
<210> 31
   <211> 20
   <212> DNA
   <213> Artificial Sequence
<220>
   <223> primer X-7
<400> 31
   tgggagtatg gagtcgacat 20
<210> 32
   <211> 21
   <212> DNA
   <213> Artificial Sequence
<220>
   <223> primer 101-23
<400> 32
   tacactaaaa tcgactcctc c 21
<210> 33
   <211> 20
   <212> DNA
   <213> Artificial Sequence
<220>
   <223> primer X-13
<400> 33
   atgagtttga gaacgatctt 20
<210> 34
   <211> 20
   <212> DNA
   <213> Artificial Sequence
<220>
   <223> primer 101-29
<400> 34
   acctgcgcct gaggctacga 20
<210> 35
   <211> 20
   <212> DNA
   <213> Artificial Sequence
<220>
   <223> primer X-17
<400> 35
   gcagatcgtt tccgtttggg 20
<210> 36
   <211> 21
   <212> DNA
   <213> Artificial Sequence
<220>
   <223> primer 101-38
<400> 36
   aacatcgaaa gattaaagaa a 21
<210> 37
   <211> 20
   <212> DNA
   <213> Artificial Sequence
<220>
   <223> primer 101-6R
<400> 37
   cacgcgattc ccatatccct 20
<210> 38
   <211> 20
   <212> DNA
   <213> Artificial Sequence
<220>
   <223> primer KIV-14
<400> 38
   cgtgtactaa ctatactgac 20
<210> 39
   <211> 20
   <212> DNA
   <213> Artificial Sequence
<220>
   <223> primer KIV-16
<400> 39
   ctcaaattcc atccgaatag 20
<210> 40
   <211> 23
   <212> DNA
   <213> Artificial Sequence
<220>
   <223> primer IA-3
<220>
   <221> misc_feature
   <222> 3, 6, 9, 12, 15
   <223> n=i
<400> 40
   ccnacnggna gnggnaarag cac 23
<210> 41
   <211> 23
   <212> DNA
   <213> Artificial Sequence
<220>
   <223> primer IV-3
<220>
   <221> misc_feature
   <222> 3, 12, 15
   <223> n=i
<400> 41
   ctnccmgtgc gnccncgscg ytg 23
<210> 42
   <211> 19
   <212> DNA
   <213> Artificial Sequence
<220>
   <223> primer III R-2
<220>
   <221> misc_feature
   <222> 3, 7, 8, 11, 14
   <223> n=i
<400> 42
   ccnggrnngt ngcngtrgc 19
<210> 43
   <211> 56
   <212> DNA
   <213> Artificial Sequence
<220>
   <223> primer 1-1F
<400> 43
   tgatctagaa tgcatcatca tcatcatcat tctcgtgaac tcgtctcgtc gtcatc 56
<210> 44
   <211> 56
   <212> DNA
   <213> Artificial Sequence
<220>
   <223> primer 1-2F
<400> 44
   tgatctagaa tgcatcatca tcatcatcat gaatggcatc tccaacccaa gatatc 56
<210> 45
   <211> 57
   <212> DNA
   <213> Artificial Sequence
<220>
   <223> primer 1-3F
<400> 45
   tgatctagaa tgcatcatca tcatcatcat aagaggaagt acaggatcca tgtagtg 57
<210> 46
   <211> 55
   <212> DNA
   <213> Artificial Sequence
<220>
   <223> primer 1-4F
<400> 46
   tgatctagaa tgcatcatca tcatcatcat cgatctgtgg tcgaggtttc atctc 55
<210> 47
   <211> 56
   <212> DNA
   <213> Artificial Sequence
<220>
   <223> primer 1-5F
<400> 47
   tgatctagaa tgcatcatca tcatcatcat aatagggaaa tgatctatac ccgcgt 56
<210> 48
   <211> 32
   <212> DNA
   <213> Artificial Sequence
<220>
   <223> primer 1-1R
<400> 48
   tcatttaaat cagagtatgc tgctgagagt tg 32
<210> 49
   <211> 30
   <212> DNA
   <213> Artificial Sequence
<220>
   <223> primer 1-2R
<400> 49
   tcatttaaat caattcggct ggagtgactg 30
<210> 50
   <211> 31
   <212> DNA
   <213> Artificial Sequence
<220>
   <223> primer 1-3R
<400> 50
   tcatttaaac tattcggagg ggggatgcga t 31
<210> 51
   <211> 32
   <212> DNA
   <213> Artificial Sequence
<220>
   <223> primer 1-4R
<400> 51
   tcatttaaat cacggaagat ggacgtacct tg 32
<210> 52
   <211> 30
   <212> DNA
   <213> Artificial Sequence
<220>
   <223> primer 1-5R
<400> 52
   tcatttaaac taaagcggtc tactctgaac 30

## Claims

1. A nucleic acid comprising the nucleotide sequence of SEQ ID NO: 1 or a nucleic acid comprising the nucleotide sequence complementary to the nucleotide sequence of SEQ ID NO: 1.

2. A primer or probe for detection of a nucleic acid according to claim 1, the primer or probe including any one of the nucleotide sequences of SEQ ID NOS: 18 to 38.

3. A method for detecting a nucleic acid according to claim 1, the method comprising using one or more primers or probes according to claim 2.

4. A method according to claim 3, wherein a combination of the polynucleotides to be used as the primers is any one of combinations of SEQ ID NOS: 18 and 19, SEQ ID NOS: 19 and 20, SEQ ID NOS: 21 and 22, SEQ ID-NOS: 23 and 24, SEQ ID NOS: 25 and 26, SEQ ID NOS: 27 and 28, SEQ ID NOS: 29 and 30, SEQ ID NOS: 31 and 32, SEQ ID NOS: 33 and 34, SEQ ID NOS: 35 and 36, and SEQ ID NOS: 37 and 38.

5. A method according to claim 4, further comprising performing a first amplification using a combination of SEQ ID NOS: 18 and 19 as a primer combination, and subsequently performing a second amplification using a combination of SEQ ID NOS: 19 and 20 as a primer combination.

6. An *in vitro* method of diagnosing hepatitis of unknown cause, the method comprising, detecting a nucleic acid according to claim 1, or a fragment thereof, in a sample obtained from a patient.

7. A diagnostic method according to claim 6, the method comprising a method according to any one of claims 3 to 5.

8. A polypeptide comprising an amino acid sequence encoded by the nucleotide sequence of nucleotides 3070 to 3669, 3826 to 4293, 5233 to 6117, 7198 to 7872, or 7888 to 8610 of SEQ ID NO: 1.

9. An antibody against the polypeptide of claim 8.

10. An *in vitro* method of diagnosing hepatitis of unknown cause, the method comprising detecting a polypeptide according to claim 8 or an antibody according to claim 9.

## Patentansprüche

1. Nukleinsäure umfassend die Nukleotidsequenz SEQ ID NO: 1 oder Nukleinsäure umfassend die zur Nukleotidsequenz SEQ ID NO: 1 komplementäre Nukleotidsequenz.

2. Primer oder Sonde zur Detektion einer Nukleinsäure nach Anspruch 1, wobei der Primer oder die Sonde eine der Nukleotidsequenzen SEQ ID NOS: 18 bis 38 enthält.

3. Verfahren zur Detektion einer Nukleinsäure nach Anspruch 1, wobei das Verfahren die Verwendung eines oder mehrerer Primer oder Sonden nach Anspruch 2 umfasst.

4. Verfahren nach Anspruch 3, wobei eine Kombination der als Primer zu verwendenden Polynukleotide eine der Kombinationen ausgewählt aus SEQ ID NOS: 18 und 19, SEQ ID NOS: 19 und 20, SEQ ID NOS: 21 und 22, SEQ ID NOS: 23 und 24, SEQ ID NOS: 25 und 26, SEQ ID NOS: 27 und 28, SEQ ID NOS: 29 und 30, SEQ ID NOS: 31 und 32, SEQ ID NOS: 33 und 34, SEQ ID NOS: 35 und 36, SEQ ID NOS: 37 und 38 ist.

5. Verfahren nach Anspruch 4, ferner umfassend Durchführen einer ersten Amplifikation mit einer Kombination der SEQ ID NOS: 18 und 19 als Primerkombination und anschließendes Durchführen einer zweiten Amplifikation mit einer Kombination der SEQ ID NOS: 19 und 20 als Primerkombination.

6. In-vitro-Verfahren zur Diagnose von Hepatitis unbekannter Ursache, das Verfahren umfassend Detektieren einer Nukleinsäure nach Anspruch 1 oder deren Fragment in einer von einem Patienten erhaltenen Probe.

7. Diagnoseverfahren nach Anspruch 6, das Verfahren umfassend ein Verfahren nach einem der Ansprüche 3 bis 5.

8. Polypeptid umfassend eine Aminosäuresequenz, die durch die Nukletidsequenz der Nukleotide 3070 bis 3669, 3826 bis 4293, 5233 bis 6117, 7198 bis 7872 oder 7888 bis 8610 der SEQ ID NO: 1 kodiert wird.

9. Antikörper gegen das Polypeptid nach Anspruch 8.

10. In-vitro-Verfahren zur Diagnose von Hepatitis unbekannter Ursache, das Verfahren umfassend Detektieren eines Polypeptids nach Anspruch 8 oder eines Antikörpers nach Anspruch 9.

## Revendications

1. Acide nucléique renfermant la séquence nucléotidique selon SEQ ID NO : 1 ou acide nucléique renfermant la séquence nucléotidique complémentaire de la séquence nucléotidique selon SEQ ID NO : 1.

2. Amorce ou sonde pour permettre la détection d'un acide nucléique conforme à la revendication 1, cette amorce où cette sonde renfermant l'une quelconque des séquences nucléotidiques selon SEQ ID NOS : 18 à 38.

3. Procédé de détection d'un acide nucléique conforme à la revendication 1, ce procédé comprenant une étape consistant à utiliser une ou plusieurs amorce(s) ou une ou plusieurs sonde(s) conforme(s) à la revendication 2.

4. Procédé conforme à la revendication 3, selon lequel une combinaison des polynucléotides destinés à être utilisés en tant qu'amorces est l'une quelconque des combinaisons selon SEQ ID NOS : 18 et 19, SEQ ID NOS : 19 et 20, SEQ ID NOS : 21 et 22, SEQ ID NOS : 23 et 24, SEQ ID NOS :25 et 26, SEQ ID NOS : 27 et 28, SEQ ID NOS : 29 et 30, SEQ ID NOS : 31 et 32, SEQ ID NOS : 33 et 34, SEQ ID NOS : 35 ET 36, et SEQ ID NOS : 37 et 38.

5. Procédé conforme à la revendication 4, comprenant en outre une étape consistant à effectuer une première amplification en utilisant une combinaison selon SEQ ID NOS : 18 et 19 en tant que combinaison d'amorce, puis à effectuer une seconde amplification en utilisant une combinaison selon SEQ ID NOS : 19 et 20 en tant que combinaison d'amorce.

6. Procédé de diagnostic in *vitro* d'une hépatite d'origine inconnue, ce procédé comprenant une étape consistant à détecter un acide nucléique conforme à la revendication 1 ou un fragment de celui-ci dans un échantillon obtenu chez un patient.

7. Procédé de diagnostic conforme à la revendication 6, ce procédé comprenant un procédé conforme à l'une quelconque des revendications 3 à 5.

8. Polypeptide comprenant une séquence d'acides aminés codée par la séquence nucléotidique des nucléotides 3070 à 3669, 3826 à 4293, 5233 à 6117, 7198 à 7872 ou 7888 à 8610 selon SEQ ID NO : 1.

9. Anticorps contre le polypeptide conforme à la revendication 8.

10. Procédé de diagnostic in *vitro* d'une hépatite d'origine inconnue, ce procédé comprenant une étape consistant à détecter un polypeptide conforme à la revendication 8 ou un anticorps conforme à la revendication 9.
